# EUROPEAN PATENT APPLICATION

(11) **EP 1 186 300 A1**
(43) Date of publication of application: **13.03.2002**
(21) Application number: 01119521.1
(22) Date of filing: 20.03.1997
(51) Int. Cl.: A61K 39/395, A61K 38/17

(54) **Pharmaceutical compositions useful for inhibiting an immune response by blocking the GP39/CD40 and CTLA4/CD28/B7 pathways**

(30) Priority: 20.03.1996 US 13751 P
(62) Divisional of application: 97916037.1
(71) Applicant: BRISTOL-MYERS SQUIBB COMPANY, Princeton, NJ 08543-4000 (US)
(72) Inventor: Larsen, Christian P., Atlanta, GA 30345 (US); Aruffo, Alejandro A., Edmonds, WA 98020 (US); Hollenbaugh, Diane L., Seattle, WA 98117 (US); Linsley, Peter S., Seattle, WA 98119 (US); Ledbetter, Jeffrey A., Seattle, WA 98117 (US); Pearson, Thomas C., Atlanta, GA 30307 (US)
(74) Representative: Kinzebach, Werner, Dr.

(57) **Abstract**

The present invention provides pharmaceutical compositions useful for inhibiting an immune response and for inhibiting rejection of transplanted tissues. This inhibition comprises preventing an endogenous molecule on a cell selected from the group consisting of gp39 and CD40 antigens from binding its endogenous ligand and preventing an endogenous molecule on a cell selected from the group consisting of CTLA4, CD28, and B7 antigens from binding its endogenous ligand. The prevention of such molecules from binding their ligand thereby blocks two independent signal pathways and inhibits the immune response resulting in transplanted tissue rejection.

## Description

Throughout this application various publications are referenced. The disclosures of these publications in their entireties are hereby incorporated by reference into this application in order to more fully describe the state of the art to which this invention pertains.

### BACKGROUND OF THE INVENTION

CD28 is expressed on most T lineage cells and plasma cells (June, C.H. et al., Immunol. Today 11, 211-16 (1990); Damle et al., Proc. Natl. Acad. Sci. 78:5096-6001 (1981)). The ligand for CD28 is B7, which is expressed on activated B cells (Linsley, P.S. et al., Proc. Natl. Acad. Sci. USA 87, 5031-35 (1990); Linsley, P.S. et al., J. Exp. Med. 173, 721-730 (1991).

CD40 is a member of the tumor necrosis factor receptor (TNFR) family of type I membrane-bound signaling receptors. Though originally identified as a B cell antigen, CD40 is expressed by all antigen presenting cells (APC) including dendritic cells, monocytes, and B cells.

The ligand for CD40 is gp39, which binds to CD40 and thus can activate B cells. Gp39 is also known as CD40L, TRAP and T-BAM. Gp39 is a type II cell surface protein with significant homology to TNF and is transiently expressed by activated T cells. In addition to T cells, gp39 is expressed by basophils, mast cells, and eesinophils.

The CD28 and CD40 pathways play essential roles in the initiation and amplification of T-dependent immune responses (Bluestone, J.A. Immunity 2, 555-9 (1995); Banchereau J., et al. Own. Rev. Immunol. 12, 881-922 (1994); Durie, F.H., et al. Science 261, 1328-30(1993); Foy, T.M., et al. J Exp Med 178, 1567-75 (1993); Van den Eertwegh, A.J.M., et al. J Exp Med 178, 1555-65 (1993)).

CD28/B7 interactions provide critical "second signals" necessary for optimal T cell activation, and IL-2 production (Jenkins, M.K., et al. J. Immunol. 147, 2461-6 (1991); Schwartz, R.H. Cell 71, 1065-8 (1992); Boussiotis, V.A., et al. J. Exp. Med. 178, 1753-1763 (1993)), whereas CD40/gp39 signals provide costimulation for B cell, macrophage, endothelial cell, and T cell activation (Grewal, I.S., et al. Nature 378, 617-620 (1995); van Essen, D., et al. Nature 378, 620-623 (1995); Hollenbaugh, D., et al. J. Exp. Med. 182, 33-40 (1995); Armitage, R.J., et al. Nature 357, 80-2 (1992); Cayabyab, M., et al. J. Immunol. 152, 1523-31 (1994); Noelle, R., et al. Proc. Natl. Acad. Sci. USA 89, 6550-6554 (1992); Alderson, M. et al. J. Exp. Med. 178, 669-674 (1993)).

Host immune responses often cause rejection of transplanted tissues and organs. Thus, inhibition of those immune responses are critical in the success of tissue transplantation. There have been studies aimed at blocking either of the CD28 or CD40 pathways, however, blockade of either of these pathways alone has not been sufficient to permit engraftment of highly immunogenic allografts (Turka, L.A., et al. Proc. NatÕl Acad. Sci. USA 89, 11102-11105 (1992); Parker, D.C., et al. Proc. NatÕl Acad. Sci. USA 92, 9560-9564 (1995); Larsen, C.P. et al. Transplantation 61, 4-9 (1996)). The monotherapies blocking either CD28 or CD40 pathway only resulted in at best temporary, and sometimes longer, periods of survival of transplanted tissues. Neither blockade alone uniformly promoted graft survival.

The vigorous immune response to xenogeneic organ transplants has served as a powerful barrier to the application of this technique to clinical transplantation (Platt J.L., Curr. Opin. Imm. 8, 721-728 (1996). Previous experimental attempts to prolong xenogeneic skin grafts have required either whole body irradiation followed by mixed xeno/syngeneic reconstitution (Ildstad S.T., Sachs D.H., Nature, 307: 168-170 (1984)), or rigorous preconditioning with thymectomy combined with depleting anti-T cell antibodies (Pierson III R.N., Winn H.J., Russell P.S., Auchincloss Jr. H., J. Exp. Med., 170:991-996 (1989); and Sharabi Y, Aksentijevich I., Sundt III T.M., Sachs D.H., Sykes M., J. Exp. Med., 172:195-202 (1990). These strategies have recently been used to promote skin graft acceptance across a discordant xenogeneic barrier (Zhao Y., Swenson K., Sergio J., Arn J.S., Sachs D.H., Sykes M., Nat. Med., 2(11):1211-1216 (1996)). However, the potential morbidity associated with cytoablative treatment regimens present a significant obstacle to the introduction of these strategies into use in clinical solid organ transplantation. Thus the development of non-cytoablative strategies to prolong xenograft survival would greatly facilitate the clinical application of these techniques.

Presently, there exists a need to provide ways to effect long-term tolerance of transplanted tissues by the host, thereby increasing the survival rate of transplantation. To do so, it is necessary to ensure sufficient immunologic unresponsiveness in the transplant recipient.

We have found that the inhibition of T-dependent immune responses resulting from blockade of either CD28 or CD40 signals is potent, but incomplete. The data herein demonstrate that simultaneous blockade of these pathways unexpectedly inhibits acute and chronic rejection of transplanted tissue in vivo. Independent blockade of these pathways using a soluble CTLA4 molecule or antibodies which recognize and bind gp39 failed to even minimally prolong survival of primary skin transplanted tissue.

The invention herein involves the discovery that simultaneous blockade of CD28 and CD40 signals promoted long-term survival of fully allogeneic as well as xenogeneic skin grafts. Prolongation of skin allograft survival was eliminated by cyclosporine A (CyA), suggesting that it is an active process requiring intact signaling via the TcR/CD3 complex and/or other CyA sensitive pathways. Moreover, CTLA4Ig/MR1 promoted long-term acceptance of primarily vascularized cardiac graft tissue, and inhibited the development of chronic vascular rejection.

The effect demonstrated in the two transplantation models herein indicates that CD28 and CD40 provide interrelated, yet independent signaling pathways required for the generation of effective T cell responses. This discovery provides methods which are new and more effective strategies to manipulate immune responses including suppressing graft rejection.

### SUMMARY OF THE INVENTION

The present invention provides a method for inhibiting rejection of a transplanted tissue. This method comprises preventing an endogenous molecule (e.g., antigen) on a cell selected from the group consisting of gp39 and CD40 from binding its endogenous ligand and preventing an endogenous molecule on a cell selected from the group consisting of CTLA4, CD28, and B7 from binding its endogenous ligand. The prevention of such molecules from binding their ligands thereby blocks two independent signal pathways and inhibits the immune response responsible for transplanted tissue rejection.

Further, the invention provides a method for inhibiting an immune response involved with transplanted tissue rejection comprising contacting a B7-positive cell with a first soluble ligand which recognizes and binds the B7 antigen, and contacting a gp39-positive cell with a second soluble ligand which recognizes and binds the gp39 antigen. The binding of the B7-positive cell to the first soluble ligand blocks the reaction of the B7 antigen with endogenous CTLA4 or CD28. Additionally, the binding of the gp39 antigen to the second soluble ligand blocks the reaction of gp39 antigen with endogenous CD40. This blockage of both the gp39 and B7 pathways inhibits immune responses.

Applicants' discovery includes a method for inhibiting immune responses mediated by the gp39 and B7 pathways in a subject. This method comprises administering to the subject a first soluble ligand which recognizes and binds the B7 antigen and a second soluble ligand which recognizes and binds the gp39 antigen.

The binding of both the first and second soluble ligands to their receptors inhibits the immune response mediated by the gp39 and B7 pathways by preventing an endogenous molecule on a cell selected from the group consisting of gp39 and CD40 antigens from binding its endogenous ligand and preventing an endogenous molecule on a cell selected from the group consisting of CTLA4, CD28, or B7 from binding its ligand.

The present invention also provides a method for inhibiting transplant rejection in a subject. This method comprises administering to the subject an effective amount of a combination of a first soluble ligand which recognizes and binds the B7 antigen on B7-positive cells and a second soluble ligand which recognizes and binds the gp39 antigen on gp39-positive cells.

The binding of B7-positive cells with the first soluble ligand and gp39-positive cells with the second soluble ligand disrupts endogenous CTLA4-, CD28-, and gp39-positive cell interactions with B7-positive cells and gp39-positive cells so that transplant rejection is inhibited.

In particular, the present invention provides:
a method for inhibiting rejection of a transplanted tissue comprising:
a) contacting a B7-positive cell with a first soluble ligand which recognizes and binds the B7 antigen, and
b) contacting a gp39-positive cell with a second soluble ligand which recognizes and binds the gp39 antigen,
the binding of the B7-positive cell to the first soluble ligand thereby blocking the reaction of the B7 antigen with endogenous CTLA4 or CD28 and the binding of the gp39 antigen to the second soluble ligand thereby blocking the reaction of gp39 antigen with endogenous CD40, the blockage thereby inhibiting the immune response; or
a method for inhibiting an immune response mediated by CTLA4-, CD28-, and gp39-positive cell interactions with B7- and CD40-positive cells in a subject comprising administering to the subject, especially an animal subject, preferably a human, a first soluble ligand which recognizes and binds the B7 antigen and a second soluble ligand which recognizes and binds the gp39 antigen, such binding thereby inhibiting the immune response mediated by CTLA4-, CD28-, and gp39-positive cell interactions with B7-positive cells and gp39-positive cells; or a method for inhibiting transplant rejection in a subject comprising administering to the subject, especially an animal subject, preferably a human, an effective amount of a combination of a first soluble ligand which recognizes and binds the B7 antigen on B7-positive cells and a second soluble ligand which recognizes and binds the gp39 antigen on gp39-positive cells, the binding of B7-positive cells with the first soluble ligand and gp39-positive cells with the second soluble ligand thereby disrupting endogenous CTLA4-, CD28-, and gp39-positive cell interactions with B7-positive cells and gp39-positive cells so that transplant rejection is inhibited.

According to one embodiment the first soluble ligand is a recombinant binding molecule having at least a portion of the extracellular domain of CTLA4, wherein the extracellular portion of CTLA4 is, according to a particular embodiment, joined to a non-CTLA4 protein sequence, e.g., at least a portion of an immunoglobulin molecule, i. e., CTLA4Ig fusion protein, e. g., CTLA4Ig designated ATCC 68629; or
a CD28Ig/CTLA4Ig fusion protein hybrid, in particular a CD28Ig/CTLA4Ig fusion protein hybrid having a first amino acid sequence corresponding to a portion of the extracellular domain of CD28 receptor fused to a second amino acid sequence corresponding to a portion of the extracellular domain of CTLA4 receptor and a third amino acid sequence corresponding to the hinge, CH2 and CH3 regions of human immunoglobulin Cg1.

According to a further embodiment, the first soluble ligand is a monoclonal antibody reactive with B7 antigen, e. g., anti-BB1 monoclonal antibody.

According to a further embodiment, the second soluble ligand for the gp39 antigen is a monoclonal antibody reactive with the gp39 antigen, e. g. MR1 monoclonal antibody.

In particular, the invention also provides a method for inhibiting an immune response comprising:
a) preventing an endogenous antigen on a cell selected from the group consisting of gp39 and CD40 from binding its endogenous ligand; and
b) preventing an endogenous antigen on a cell selected from the group consisting of CTLA4, CD28, and B7 from binding its endogenous ligand,
the prevention of such antigens from binding their ligand thereby blocking two independent cell signals and inhibiting the immune response,
wherein, according to one embodiment
a) the step of preventing the endogenous gp39 antigen from binding its endogenous ligand comprises contacting a gp39-positive cell with a soluble ligand which recognizes and binds the gp39 antigen, in particular with a monoclonal antibody reactive with the gp39 antigen,
b) the step of preventing the endogenous CTLA4 antigen from binding its endogenous ligand comprises contacting a B7-positive cell with a soluble ligand which recognizes and binds the B7 antigen, in particular with a CTLA4Ig,
the binding of the gp39-positive cell to its soluble ligand of step (a) thereby blocking the reaction of endogenous gp39 antigen with endogenous CD40, the binding of the B7-positive cell to its soluble ligand of step (b) thereby blocking the reaction of the endogenous B7 antigen with endogenous CTLA4, the blockage thereby inhibiting the immune response; or
wherein, according to a further embodiment,
a) the step of preventing the endogenous CD40 antigen from binding its endogenous ligand comprises contacting a CD40-positive cell with a soluble ligand which recognizes and binds the CD40 antigen, in particular with a monoclonal antibody directed against CD40,
b) the step of preventing the endogenous CTLA4 antigen from binding its endogenous ligand comprises contacting a B7-positive cell with a soluble ligand which recognizes and binds the B7 antigen, in particular with a CTLA4Ig,
the binding of the CD40-positive cell to its soluble ligand of step (a) thereby blocking the reaction of endogenous CD40 antigen with endogenous gp39, the binding of the B7-positive cell to its soluble ligand of step (b) thereby blocking the reaction of the B7 antigen with endogenous CTLA4, the blockage thereby inhibiting the immune response; or
wherein, according to a further embodiment,
a) the step of preventing the endogenous gp39 antigen from binding its endogenous ligand comprises contacting a gp39-positive cell with a soluble ligand which recognizes and binds the gp39 antigen, in particular with a monoclonal antibody reactive with the gp39 antigen,
b) the step of preventing the endogenous CD28 antigen from binding its endogenous ligand comprises contacting a B7-positive cell with a soluble ligand which recognizes and binds the B7 antigen, in particular with a CTLA4Ig,
the binding of the gp39-positive cell to its soluble ligand of step (a) thereby blocking the reaction of gp39 antigen with endogenous CD40, the binding of the B7-positive cell to its soluble ligand of step (b) thereby blocking the reaction of the B7 antigen with endogenous CD28, the blockage thereby inhibiting the immune response; or
wherein, according to a further embodiment,
a) the step of preventing the endogenous CD40 antigen from binding its endogenous ligand comprises contacting a CD40-positive cell with a soluble ligand which recognizes and binds the CD40 antigen, in particular with a monoclonal antibody directed against CD40,
b) the step of preventing the endogenous B7 antigen from binding its endogenous ligand comprises contacting a CD28-positive cell with a soluble ligand which recognizes and binds the CD28 antigen, in particular with a monoclonal antibody directed against CD28,
the binding of the CD40-positive cell to the soluble ligand of step (a) thereby blocking the reaction of CD40 antigen with endogenous gp39, the binding of the CD28-positive cell to the soluble ligand of step (b) thereby blocking the reaction of the CD28 antigen with endogenous B7, the blockage thereby inhibiting the immune response; or
wherein, according to a further embodiment,
a) the step of preventing the endogenous CD40 antigen from binding its endogenous ligand comprises contacting a CD40-positive cell with a soluble ligand which recognizes and binds the CD40 antigen, in particular with a sgp39,
b) the step of preventing the endogenous B7 antigen from binding its endogenous ligand comprises contacting a CTLA4-positive cell with a soluble ligand which recognizes and binds the CTLA4 antigen, in particular with a monoclonal antibody directed against CTLA4,
the binding of the CD40-positive cell to the soluble ligand of step (a) thereby blocking the reaction of CD40 antigen with endogenous gp39, the binding of the CTLA4- or CD28-positive cell to the soluble ligand of step (b) thereby blocking the reaction of the CTLA4 antigen with endogenous B7, the blockage thereby inhibiting the immune response; or
wherein, according to a further embodiment,
a) the step of preventing the endogenous CD40 antigen form binding its endogenous ligand comprises contacting a gp39-positive cell with a soluble ligand which recognizes and binds the gp39 antigen, in particular with a monoclonal antibody reactive with the gp39 antigen,
b) the step of preventing the endogenous B7 antigen from binding its endogenous ligand comprises contacting a CD28-possitive cell with a soluble ligand which recognizes and binds the CD28 antigen, in particular with a monoclonal antibody directed against CD28,
the binding of the gp40-positive cell to the soluble ligand of step (a) thereby blocking the reaction of gp39 antigen with endogenous CD40, the binding of the CD28-positive cell to the soluble ligand of step (b) thereby blocking the reaction of the CD28 antigen with endogenous B7, the blockage thereby inhibiting the immune response; or
wherein, according to a further embodiment,
a) the step of preventing the endogenous CD40 antigen from binding its endogenous ligand comprises contacting a gp39-positive cell with a soluble ligand which recognizes and binds the gp39 antigen, in particular with a monoclonal antibody reactive with the gp39 antigen,
b) the step of preventing the endogenous B7 antigen from binding its endogenous ligand comprises contacting a CTLA4-positive cell with a soluble ligand which recognizes and binds the CTLA4 antigen, in particular with a monoclonal antibody directed against CTLA4,
the binding of the CD40-positive cell to the soluble ligand of step (a) thereby blocking the reaction of gp39 antigen with endogenous CD40, the binding of the CTLA4-positive cell to the soluble ligand of step (b) thereby blocking the reaction of the CTLA4 antigen with endogenous B7, the blockage thereby inhibiting the immune response.

Further, the invention provides a pharmaceutical composition useful to inhibit an immune response comprising a pharmaceutically effective amount of a soluble ligand which recognizes and binds a B7 antigen and an acceptable carrier;
a pharmaceutical composition useful to inhibit an immune response comprising a pharmaceutically effective amount of a soluble ligand which recognizes and binds a CD28 antigen and an acceptable carrier;
a pharmaceutical composition useful to inhibit an immune response comprising a pharmaceutically effective amount of a soluble ligand which recognizes and binds a CTLA4 antigen and an acceptable carrier;
a pharmaceutical composition useful to inhibit an immune response comprising a pharmaceutically effective amount of a soluble ligand which recognizes and binds a gp39 antigen and an acceptable carrier;
a pharmaceutical composition useful to inhibit an immune response comprising a pharmaceutically effective amount of a soluble ligand which recognizes and binds a CD40 antigen and an acceptable carrier;
a pharmaceutical composition useful to inhibit an immune response comprising a pharmaceutically effective amount of a combination of a soluble ligand which recognizes and binds a B7 antigen and a soluble ligand which recognizes and binds a gp39 antigen and an acceptable carrier;
a pharmaceutical composition useful to inhibit an immune response comprising a pharmaceutically effective amount of a combination of a soluble ligand which recognizes and binds a CD28 antigen and a soluble ligand which recognizes and binds a CD40 antigen and an acceptable carrier;
a pharmaceutical composition useful to inhibit an immune response comprising a pharmaceutically effective amount of a combination of a soluble ligand which recognizes and binds a CTLA4 antigen and a soluble ligand which recognizes and binds a CD40 antigen and an acceptable carrier;
a pharmaceutical composition useful to inhibit and immune response comprising a pharmaceutically effective amount of a combination of a soluble ligand which recognizes and binds a gp39 antigen and a soluble ligand which recognizes and binds a CD28 antigen and an acceptable carrier;
a pharmaceutical composition useful to inhibit and immune response comprising a pharmaceutically effective amount of a combination of a soluble ligand which recognizes and binds a gp39 antigen and a soluble ligand which recognizes and binds a CTLA4 antigen and an acceptable carrier;
a pharmaceutical composition useful to inhibit an immune response comprising a pharmaceutically effective amount of a combination of a soluble ligand which recognizes and binds a B7 antigen and a soluble ligand which recognizes and binds a CD40 antigen and an acceptable carrier; and
a method of inhibiting an immune response in a subject comprising administering to the subject an amount of said pharmaceutical compositions effective to inhibit the immune response in the subject.

### BRIEF DESCRIPTION OF THE FIGURES

Figure 1 is a bar graph showing that simultaneous blockade of CD28 and CD40 signals ablate popliteal lymph node alloimmune responses in vivo.

Figure 2A is a line graph that shows CTLA4Ig/MR1 treatment prolongs cardiac allograft survival in comparison with CTLA4Ig or MR1 alone.

Figure 2B is a photograph of a histologic section showing CTLA4Ig-treated cardiac allograft at day 62 having extensive lymphocytic infiltration, interstitial fibrosis, and severe coronary arterial intimal thickening and fibrosis consistent with chronic rejection (left panel 100X magnification; right panel 400X magnification).

Figure 2C is a photograph of a histologic section showing a MR1-treated cardiac allograft at day 62 having less lymphocytic infiltration and interstitial fibrosis, but severe coronary vasculopathy characteristic of chronic rejection (left panel 100X magnification; right panel 400X magnification).

Figure 2D is a photograph of a histologic section showing CTLA4Ig/MR1-treated cardiac allografts at day 58, free from lymphocytic infiltration, fibrosis, coronary arterial intimal lesions (left panel 100X magnification; right panel 400X magnification).

Figure 2E is a photograph of a histologic section showing normal untransplanted BALB/c hearts (left panel 100X magnification; right panel 400X magnification).

Figure 3A is a photograph of ethidium bromide stained gel strips showing intragraft expression of immune mediator transcripts using RT-PCR in untreated, MR1 treated, CTLA4Ig treated, and MR1/CTLA4Ig treated cardiac allografts.

Figure 3B is a series of bar graphs showing the mean PCR product band intensities ± standard deviation.

Figure 4A is a line graph showing data of mice treated with MR1 alone, CTLA4Ig alone, and a combination of MR1 and CTLA4Ig.

Figure 4B is a line graph showing data of mice treated with CyA, CyA and CTLA4Ig, and CyA and MR1.

Figure 4C is a line graph showing the effects of perioperative treatment with YTS191 and MR1, alone, MR1 and CTLA4Ig, and YTS191 and CTLA4Ig on primary skin allografts.

Figure 4D is a photograph showing healthy appearance of a BALB/c skin graft on a CTLA4Ig/MR1 treated recipient.

Figure 4E is a photograph showing a control allograft undergoing rejection.

Figure 4F is a photograph of a histologic section of a skin graft showing healthy appearance of an accepted graft at 100 days after transplant showing well preserved epidermis hair follicles and adnexal structures.

Figure 4G is a photograph showing a BALB/c skin graft on an untreated recipient eight days after transplant. The graft shows extensive lymphocytic infiltrate.

Figure 5A is a series of line graphs showing the effects in vitro of using MR1 alone, CTLA4Ig alone, and a combination of MR1/CTLA4Ig on three different cell populations.

Figure 5B is a series of bar graphs showing the effects in vivo of using MR1 alone, CTLA4Ig alone, and a combination of MR1/CTLA4Ig.

Figure 6A is a bar graph showing weight of the immunized popliteal lymph node relative to the contralateral node in C3H mice in response to foot pad immunization with irradiated (2000 RADS) rat (Sprague-Dawley) splenocytes. Human IgG (stippled), CTLA4-Ig (gray), MR1 (white), CTLA4-Ig/MR1 (black), normal unimmunized node (hatched).

Figure 6B is a line graph showing in vitro proliferation of lymph node cells after harvesting the popliteal lymph at five days after immunization. Human IgG (stippled), CTLA4-Ig (gray), MR1 (white), CTLA4-Ig/MR1 (black), normal unimmunized node (hatched).

Figure 6C is a bar graph showing that simultaneous blockade of the CD40 and CD28 pathways markedly inhibits cytokine production of IL-2. Human IgG (stippled), CTLA4-Ig (gray), MR1 (white), CTLA4-Ig/MR1 (black), normal unimmunized node (hatched).

Figure 6D is a bar graph showing that simultaneous blockade of the CD40 and CD28 pathways markedly inhibits cytokine production of INFg. Human IgG (stippled), CTLA4-Ig (gray), MR1 (white), CTLA4-Ig/MR1 (black), normal unimmunized node (hatched).

Figure 7A is a line graph showing that C3H recipients treated with CTLA4-Ig (500 µg) on days 0, 2, 4 and 6 combined with MR1 (500 µg) on days 0, 2, 4 and 6 had prolonged survival of Sprague-Dawley rat cardiac allografts.

Figure 7B is a photograph of an untreated cardiac xenograft at day 6 showing widespread tissue destruction (400X).

Figure 7C is a photograph of a CTLA4-Ig treated cardiac xenograft at day 20 showing lymphocytic infiltration, myocyte destruction, and coronary vasculopathy (400X).

Figure 7D is a photograph of a MR1 treated cardiac xenograft at day 20 showing lymphocytic infiltration, myocyte destruction, and coronary vasculopathy (400X).

Figure 7E is a photograph of a normal imtransplanted Sprague-Dawley rat heart (400X).

Figure 7F is a photograph of a CTLA4-Ig/MR1 treated cardiac xenograft at day 20, essentially free from lymphocytic infiltration and fibrosis (400X).

Figure 7G is a photograph of a CTLA4-Ig/MR1 treated cardiac xenograft at day 122, demonstrating excellent preservation of both myocytes and vascular structures (400X).

Figure 8A is a series of line graphs showing prolongation of Sprague-Dawley rat skin xenograft survival in C3H mice treated with MR1 and CTLA4-Ig administered together in the perioperative period as compared with xenograft recipients treated with either MR1 alone or CTLA4-Ig alone and untreated controls.

Figure 8B is a series of line graphs showing no significant change in xenograft survival following chronic treatment (beginning after the standard 4 dose regimen) with either the CTLA4-Ig/MR1 combination or MR1.

Figure 8C is a photograph showing the healthy appearance of a Sprague-Dawley rat skin graft on a CTLA4-Ig/MR1 treated C3H recipient at 100 days after transplant.

Figure 8D is a photograph showing a control xenograft of skin undergoing rejection at 10 days post transplant.

Figure 8E is a photograph of a hematoxylin-eosin stained histologic section of an accepted CTLA4-Ig/MR1 treated graft at 50 days after transplant showing well-preserved histologic architecture (400X).

Figure 8F is a photograph of a hematoxylin-eosin stained histologic section of a Sprague-Dawley rat skin graft on an untreated C3H recipient 8 days after transplant showing extensive lymphocytic infiltrates (400X).

Figure 9A is a scatter plot showing ablation of evoked xenoantibody response in serum collected from C3H recipients 55 days after skin xenografts from Sprague-Dawley donors. Control mice that had received no treatment had readily detectable IgG xenoantibody. Either CTLA4-Ig or MR1 alone partially blocked the xenoantibody response. The combination of CTLA4-Ig and MR1 essentially ablated the evoked xenoantibody response. Each data point represents the analysis of an individual recipient.

Figure 9B is a scatter plot showing ablation of evoked xenoantibody response in serum collected from C3H recipients 20 days after heart xenografts from Sprague-Dawley donors.

### DETAILED DESCRIPTION OF THE INVENTION

### DEFINITIONS

All scientific and technical terms used in this application have meanings commonly used in the art unless otherwise specified. As used in this application, the following words or phrases have the meanings specified.

As used herein "monoclonal antibodies directed against gp39" or "anti-gp39" includes MR1. Anti-gp39 is also known in the literature as an antiCD40 ligand. Examples of MR1 include, but are not limited to monoclonal antibodies directed against gp39 from mouse; antibodies directed against gp39 from other species such as monkey, sheep, human are included. Additionally, "monoclonal antibodies directed against gp39" or "anti-gp39" includes any antibody molecule, fragment thereof, or recombinant binding protein that recognizes and binds gp39.

As used herein, "administering" means oral administration, administration as a suppository, topical contact, intravenous, intraperitoneal, intramuscular or subcutaneous administration, or the implantation of a slow-release device such as a miniosmotic pump, to the subject.

As used herein, "pharmaceutically acceptable carrier" includes any material which when combined with the antibody retains the antibody's immunogenicity and is nonreactive with the subject's immune systems. Examples include, but are not limited to, any of the standard pharmaceutical carriers such as a phosphate buffered saline solution, water, emulsions such as oil/water emulsion, and various types of wetting agents. Other carriers may also include sterile solutions, tablets including coated tablets and capsules.

Typically such carriers contain excipients such as starch, milk, sugar, certain types of clay, gelatin, stearic acid or salts thereof, magnesium or calcium stearate, talc, vegetable fats or oils, gums, glycols, or other known excipients. Such carriers may also include flavor and color additives or other ingredients. Compositions comprising such carriers are formulated by well known conventional methods.

As used herein, "transplanted tissue" includes autografts, isografts, allografts, and xenografts. Examples of transplanted tissue include, but are not limited to, solid organ transplants such as heart, liver or kidney, skin grafts, pancreatic islet cells, bone marrow grafts or cell suspensions.

As used herein, "B7" includes B7-1 (also called CD80), B7-2 (also called CD86), B7-3, and the B7 family, e.g., a combination of B7-1, B7-2 and/or B7-3.

In order that the invention herein described may be more fully understood, the following description is set forth.

The discovery herein is related to a method for inhibiting rejection of a transplanted tissue. In one embodiment, the method comprises preventing an endogenous molecule on a cell selected from the group consisting of gp39 and CD40 from binding its endogenous ligand. The method provides preventing an endogenous molecule on a cell selected from the group consisting of CTLA4, CD28, and B7 from binding its endogenous ligand. The prevention of these molecules from binding their endogenous ligands blocks two independent signal pathways. The blockage of these two independent signal pathways inhibits the immune responses that cause transplanted tissue rejection.

In one example of the invention, endogenous gp39 antigen is prevented from binding its endogenous ligand. This example comprises the step of contacting a gp39-positive cell with a soluble ligand which recognizes and binds the gp39 antigen (e.g., by using soluble ligands such as MR1 or other antibodies which bind gp39, and soluble CD40 molecules).

This example comprises the additional step of preventing the endogenous CTLA4 antigen from binding its endogenous ligand. This comprises the step of contacting a B7-positive cell with a soluble ligand which recognizes and binds the B7 antigen such as CTLA4Ig (United States Patent No. 5,434,131, issued July 18, 1995), the BB-1 monoclonal antibody or other antibodies directed against B7.

The binding of the gp39-positive cell to its soluble ligand blocks the reaction of endogenous gp39 antigen with endogenous CD40. The binding of the B7-positive cell to its soluble ligand blocks the reaction of the endogenous B7 antigen with endogenous CTLA4 and CD28. This combined blockage inhibits the immune response.

In another example, endogenous CD40 antigen is prevented from binding its endogenous ligand. This example comprises the step of contacting a CD40-positive cell with a soluble ligand which recognizes and binds the CD40 antigen. Suitable ligands include antibodies directed against CD40 or soluble gp39 (sgp39).

This example comprises the additional step of preventing the endogenous CTLA4 antigen from binding its endogenous ligand. This step comprises contacting a B7-positive cell with a soluble ligand which recognizes and binds the B7 antigen. Examples of this soluble ligand include CTLA4Ig, soluble CD28 molecules, and antibodies directed against B7.

The binding of the CD40-positive cell to its soluble ligand blocks the reaction of endogenous CD40 antigen with endogenous gp39. The binding of the B7-positive cell to its soluble ligand blocks the reaction of the B7 antigen with endogenous CTLA4. The combined blockage inhibits the immune response.

In yet another example, endogenous gp39 antigen is prevented from binding its endogenous ligand as described above. The example comprises the additional step of preventing the endogenous CD28 antigen from binding its endogenous ligand. This step comprises contacting a B7-positive cell with a soluble ligand which recognizes and binds the B7 antigen. Examples include CTLA4Ig, soluble CD28 molecules, and antibodies directed against B7 such as BB-1.

The binding of the gp39-positive cell to its soluble ligand blocks the reaction of gp39 antigen with endogenous CD40. The binding of the B7-positive cell to its soluble ligand blocks the reaction of the B7 antigen with endogenous CD28. This combined blockage inhibits the immune response.

In another example, endogenous CD40 antigen is prevented from binding its endogenous ligand as described above. The example provides the additional step of preventing the endogenous B7 antigen from binding its endogenous ligand. This comprises contacting a CD28-positive cell with a soluble ligand which recognizes and binds the CD28 antigen. Examples of such soluble ligands include soluble B7 molecules and antibodies directed against CD28.

The binding of the CD40-positive cell to the soluble ligand blocks the reaction of CD40 antigen with endogenous gp39. The binding of the CD28-positive cell to the soluble ligand blocks the reaction of the B7 antigen with endogenous CD28. This combined blockage inhibits the immune response.

In yet another example, endogenous CD40 antigen is prevented from binding its ligand as described above. This example provides the additional step of preventing the endogenous B7 antigen from binding its endogenous ligand which comprises contacting a CTLA4-positive cell with a soluble ligand which recognizes and binds the CTLA4 antigen. Examples of such soluble ligands include soluble B7 molecules and antibodies directed against CTLA4.

The binding of the CD40-positive cell to the soluble ligand blocks the reaction of CD40 antigen with endogenous gp39. Additionally, the binding of the CTLA4- or CD28-positive cell to the soluble ligand blocks the reaction of the CTLA4 antigen with endogenous B7. This combined blockage inhibits the immune response.

In a further example, endogenous CD40 antigen is prevented from binding its ligand as described above. This example provides the additional step of preventing the endogenous B7 antigen from binding its endogenous ligand which comprises contacting a CD28-positive cell with a soluble ligand which recognizes and binds the CD28 antigen. Examples of such soluble ligands include soluble B7 molecules and antibodies directed against CD28.

The binding of the CD40-positive cell to the soluble ligand blocks the reaction of gp39 antigen with endogenous CD40. Further, the binding of the CD28-positive cell to the soluble ligand blocks the reaction of the CD28 antigen with endogenous B7. This combined blockage inhibits the immune response.

Also, in another example, endogenous CD40 antigen is prevented from binding its ligand as described above. This example provides the additional step of preventing the endogenous B7 antigen from binding its endogenous ligand which comprises contacting a CTLA4-positive cell with a soluble ligand which recognizes and binds the CTLA4 antigen.

The binding of the CD40-positive cell to the soluble ligand blocks the reaction of gp39 antigen with endogenous CD40. Additionally, the binding of the CTLA4-pesitive cell to the soluble ligand blocks the reaction of the CTLA4 antigen with endogenous B7. This combined blockage inhibits the immune response.

Additionally, the present invention provides another embodiment for a method for inhibiting an immune response resulting in graft rejection. This embodiment comprises contacting a B7-positive cell with a first soluble ligand which recognizes and binds the B7 antigen, and contacting a gp39-positive cell with a second soluble ligand which recognizes and binds the gp39 antigen.

The binding of the B7-positive cell to the first soluble ligand blocks the reaction of the B7 antigen with endogenous CTLA4 or CD28. Further, the binding of the gp39 antigen to the second soluble ligand blocks the reaction of gp39 antigen with endogenous CD40. The combination of this blockage inhibits the immune response.

Additionally, the invention provides a method for inhibiting an immune response mediated by the CTLA4/CD28/B7 and gp39/CD40 pathways in a subject. In accordance with the practice of the invention, the subject may be an animal subject such as a human, a dog, a cat, a sheep, a horse, a mouse, a pig, or a cow.

The method comprises administering to the subject a first soluble ligand which recognizes and binds the B7 antigen (e.g. soluble CTLA4 or CD28 molecules) and a second soluble ligand which recognizes and binds the gp39 antigen (e.g., monoclonal antibodies directed against gp39 (MR1) or soluble CD40 molecules). The binding of the first and second ligands to their receptor inhibits the immune response mediated by CTLA4-, CD28-, and gp39- cell interactions with B7- and CD40-positive cells.

Also, the invention provides a method for inhibiting transplant rejection in a subject. This method comprises administering to the subject an effective amount of a combination of a first soluble ligand which recognizes and binds the B7 antigen on B7-positive cells and a second soluble ligand which recognizes and binds the gp39 antigen on gp39-positive cells. The binding of B7-positive cells with the first soluble ligand and gp39-positive cells with the second soluble ligand disrupts endogenous CTLA4-, CD28-, and gp39- cell interactions with B7-positive cells and gp39-positive cells so that transplant rejection is inhibited.

In accordance with the practice of the invention, the first soluble ligand may be a recombinant binding molecule having at least a portion of the extracellular domain of CTLA4. In accordance with the practice of the invention, the extracellular portion of CTLA4 is joined to a non-CTLA4 protein sequence. The non-CTLA4 protein sequence may be at least a portion of an immunoglobulin molecule.

In one specific example of the invention, the ligand is CTLA4Ig fusion protein, e.g., the CTLA4Ig fusion protein deposited with the American Type Culture Collection (ATCC) in Rockville, Maryland, under the provisions of the Budapest Treaty on May 31, 1991 and accorded ATCC accession number: 68629. Alternatively, the ligand may be a CD28Ig/CTLA4Ig fusion protein hybrid (United States Patent No. 5,434,131, issued July 18, 1995).

In an alternative embodiment, the first soluble ligand may be a monoclonal antibody reactive with B7 antigen, e.g., the antibody may be anti-BB1 monoclonal antibody (Clark et al., Human Immunol. 16:100-113 (1986); Yokochi et al., J. Immunol. 128:823 (1981)); Freeman et al. (J. Immunol. 143(8):2714-2722 (1989); and Freedman et al., J. Immunol. 139:3260 (1987)).

In another embodiment, the ligand may be a CD28Ig/CTLA4Ig fusion protein hybrid having a first amino acid sequence corresponding to a portion of the extracellular domain of CD28 receptor fused to a second amino acid sequence corresponding to a portion of the extracellular domain of CTLA4 receptor and a third amino acid sequence corresponding to the hinge, CH2 and CH3 regions of human immunoglobulin Cg1.

In one embodiment of the invention, the second soluble ligand for the gp39 antigen may be a monoclonal antibody reactive with the gp39 antigen, e.g., the MR1 anti-murine monoclonal antibody or the anti-human gp39 antibody (United States Patent No. 5,474,771, issued December 12, 1995).

In another embodiment of the invention, the method comprises administering to the subject a soluble fusion protein, the soluble fusion protein comprising a first binding domain and a second binding domain.

In one example, the first binding domain is a ligand which recognizes and binds the gp39 antigen. Examples include CD40 and monoclonal antibodies directed against gp39. In another example, the first binding domain is a ligand which recognizes and binds the CD40 antigen. Examples include gp39 and monoclonal antibodies directed against CD40.

In one example, the second binding domain is a ligand which recognizes and binds CTLA4. Examples include B7 and monoclonal antibodies directed against CTLA4. In another example, the second binding domain is a ligand which recognizes and binds the CD28 antigen. Examples include B7 and monoclonal antibodies directed against CD28. In another example, the second binding domain is a ligand which recognizes and binds the B7 antigen. Examples include CTLA4, CD28 and monoclonal antibodies directed against B7.

Soluble ligands may be administered during transplant, before transplant, or after transplant. Soluble ligands may be administered by oral means, transdermal means, intravenous means, intramuscular means, intraperitoneal, or by subcutaneous administration.

The most effective mode of administration and dosage regimen for the molecules of the present invention depends upon the location of the tissue or disease being treated, the severity and course of the medical disorder, the subject's health and response to treatment and the judgment of the treating physician. Accordingly, the dosages of the molecules should be titrated to the individual subject.

By way of example, the interrelationship of dosages for animals of various sizes and species and humans based on mg/m2 of surface area is described by Freireich, E.J., et al. Cancer Chemother., Rep. 50 (4): 219-244 (1966). Adjustments in the dosage regimen may be made to optimize suppression of the immune response resulting in graft rejection, e.g., doses may be divided and administered on a daily basis or the dose reduced proportionally depending upon the situation (e.g., several divided doses may be administered daily or proportionally reduced depending on the specific therapeutic situation).

It would be clear that the dose of the composition of the invention required to achieve an appropriate clinical outcome may be further reduced with schedule optimization.

The present invention also provides pharmaceutical compositions useful in inhibiting graft rejection or in inhibiting an immune response. In one embodiment, these compositions comprise an effective amount of a combination of (a) soluble ligands which recognize and bind any one of CTLA4, CD28, and B7 antigens, together with (b) soluble ligands which recognize and bind any one of gp39 and CD40 antigens and an acceptable carrier. In another embodiment, these compositions comprise an effective amount of a soluble fusion protein comprising a first binding domain and a second binding domain, wherein the first binding domain is a ligand which recognizes and binds any one of gp39 or CD40 antigens and the second binding domain is a ligand which recognizes and binds any one of CTLA4, CD28, and B7 antigens.

ADVANTAGES OF THE INVENTION: Despite the many advances in clinical immunosuppression, chronic vascular rejection remains the major source of transplant failure for which there remains no effective therapy. The experiments described herein show that blocking the CD28/CTLA4/B7 and gp39/CD40 pathways inhibits the development of chronic transplant vasculopathy in transplanted tissues. These data show that immune responses to allogeneic and xenogeneic grafts can be inhibited without cytoablation. When compared to the use of soluble CTLA4 molecules alone, the use of soluble CTLA4 molecules together with a soluble ligand that recognizes and binds gp39 provides dramatically prolonged immunosuppression.

The following examples are presented to illustrate the present invention and to assist one of ordinary skill in making and using the same. The examples are not intended in any way to otherwise limit the scope of the invention.

### EXAMPLE 1

The data in this example show that simultaneous blockade of CD28 and CD40 signals ablates popliteal lymph node alloimmune responses in vivo.

### METHOD

Male C3H/HeJ (The Jackson Laboratory, Bar Harbor, ME) mice were subcutaneously immunized with 2 x 106 BALB/c splenocytes in 50 ml of sterile normal saline in the left foot pad and 50 ml of sterile normal saline in the right foot pad on day 0 and then treated intraperitoneally with MR1 (250 mg), CTLA4Ig (250 mg), or both reagents on days 0,2 and 4.

The mice were sacrificed on day 5, the popliteal lymph nodes were harvested using an operating microscope (20X magnification) and the fresh weight of each node was determined to the nearest 0.1 mg with an analytical balance (Model A-160, Denver Instrument Company, Arvada, CO).

### DISCUSSION

Five days after subcutaneous immunization with allogeneic splenocytes, the draining popliteal lymph nodes on the side of antigen challenge underwent a >5 fold increase in weight relative to the contralateral node in untreated control mice. Treatment with either CTLA4Ig or MR1 resulted in a 50-60% inhibition of the response, whereas concomitant administration of CTLA4Ig and MR1 ablated lymph node expansion in response to antigen challenge. The results represent the mean ± standard deviation for 3 individual mice in each group. Similar results were obtained in three independent experiments.

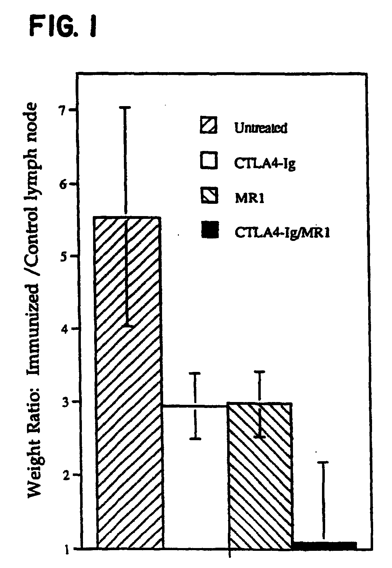

Control mice demonstrated a 4-6 fold increase in the weight of the node draining the immunized foot relative to the node draining the contralateral foot injected with sterile saline (Figure 1). This increase in weight was accompanied by a dramatic expansion of the lymphocyte-rich paracortical (T cell) and cortical (B cell) regions. When administered alone, CTLA4Ig and MR1 each produced partial inhibition of this response (57% and 56% inhibition, respectively). The combination of CTLA4Ig/MR1 ablated lymph node expansion (98% inhibition, Figure 1) and prevented expansion of the paracortical and lymphoid follicles.

### EXAMPLE 2

This example shows prolongation of cardiac allograft survival and inhibition of vasculopathy associated with chronic rejection.

### METHOD

Male C3H/HeJ mice were transplanted with primarily vascularized BALB/c heart allografts at 8-12 weeks of age using microsurgical techniques (Corry, R.J., Winn, H.J. & Russell, P.S. Transplantation 16, 343-350 (1973)).

Rejection was defined by the loss of palpable cardiac contractions with confirmation at laparotomy by direct visualization. At specified times after transplant, the transplanted hearts were excised, formalin fixed and embedded in paraffin. Tissue sections (5 mm) were stained with Masson's Trichrome or hematoxylin-eosin. Each histologic specimen was reviewed by a cardiac transplant pathologist (KJW) blinded to the treatment modality.

### DISCUSSION

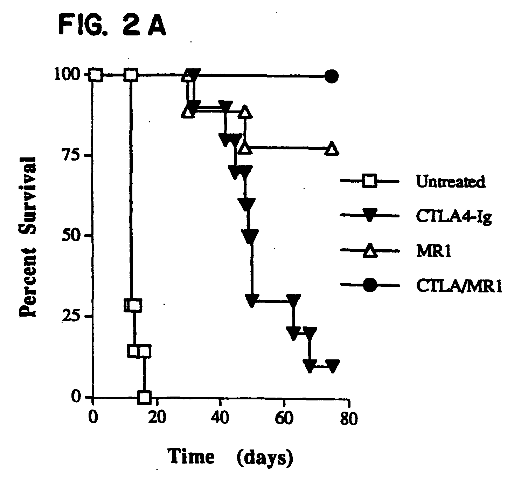

In Figure 2A C3H/HeJ recipients were treated with CTLA4Ig (200 mg/dose) on days 0, 2, 4 and 6 combined with MR1 (250 mg/dose) on days 0, 2 and 4, and had long term survival of BALB/c cardiac allografts (Median Survival Time (MST) >70 days, n = 7). The control groups included recipients treated with: CTLA4Ig alone (MST = 50 days, n = 12); MR1 alone (MST = 70 days, n = 12); and no treatment (MST = 12 days, n = 7).

All recipients were followed for 70 days with the exception of three mice with surviving transplants in each experimental group which were sacrificed for histologic analysis at 58-63 days post transplant.

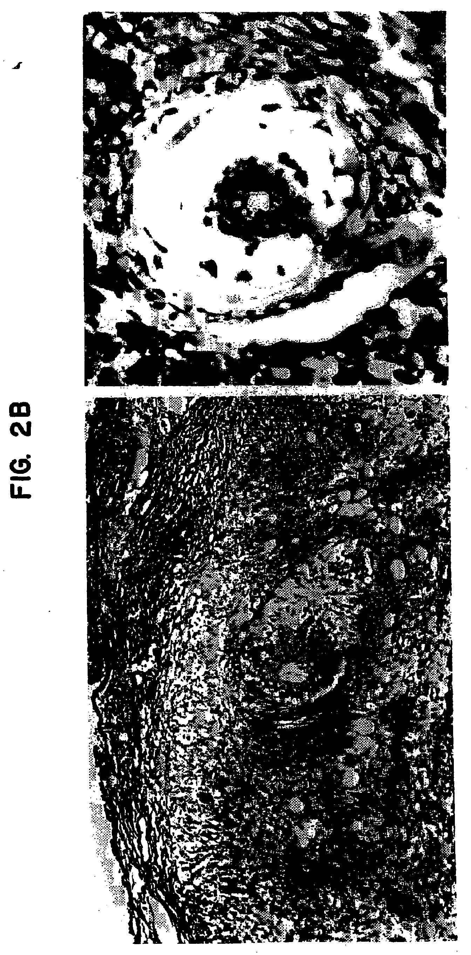

In Figure 2B, CTLA4Ig-treated cardiac allograft at day 62 shows extensive lymphocytic infiltration, interstitial fibrosis, and severe coronary arterial intimal thickening and fibrosis consistent with chronic rejection.

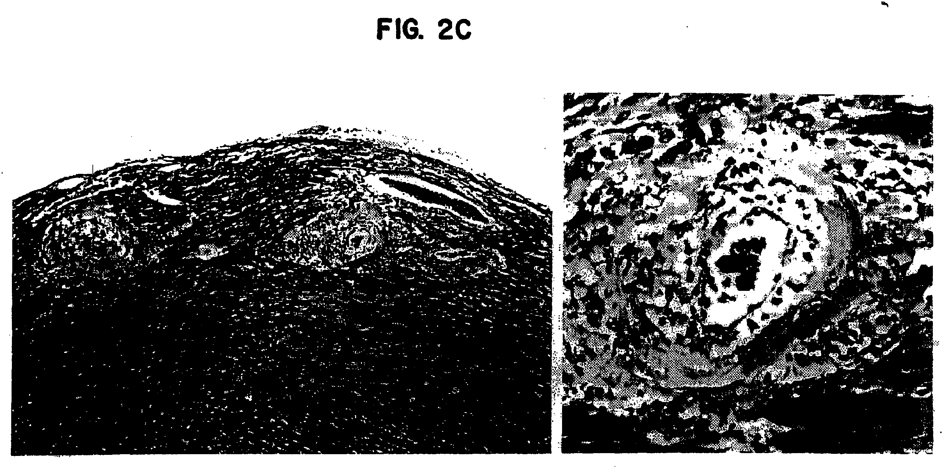

In-Figure 2C, MR1-treated cardiac allograft at day 62 demonstrates less lymphocytic infiltration and interstitial fibrosis, but severe coronary vasculopathy characteristic of chronic rejection.

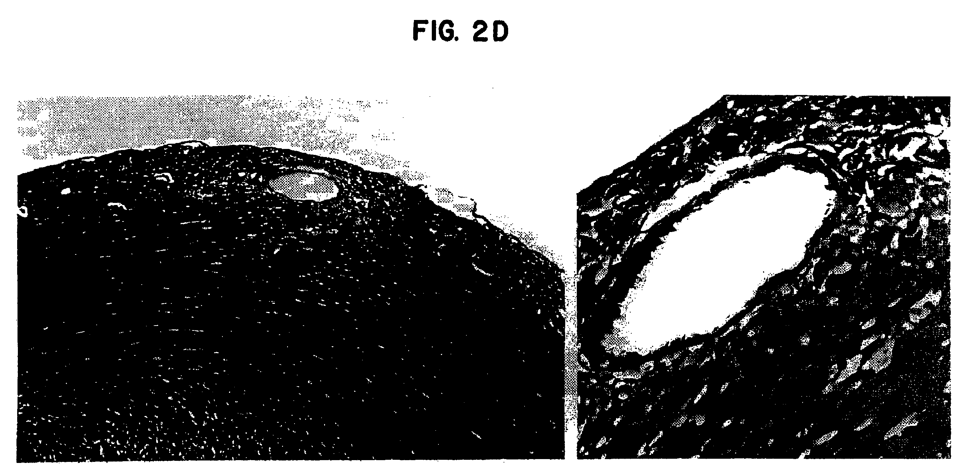

In Figure 2D, CTLA4Ig/MR1-treated cardiac allografts at day 58, in marked contrast, were remarkably free from lymphocytic infiltration, fibrosis, and most significantly, coronary arterial intimal lesions. The parenchyma and blood vessels of these grafts were virtually indistinguishable from normal untransplanted BALB/c hearts.

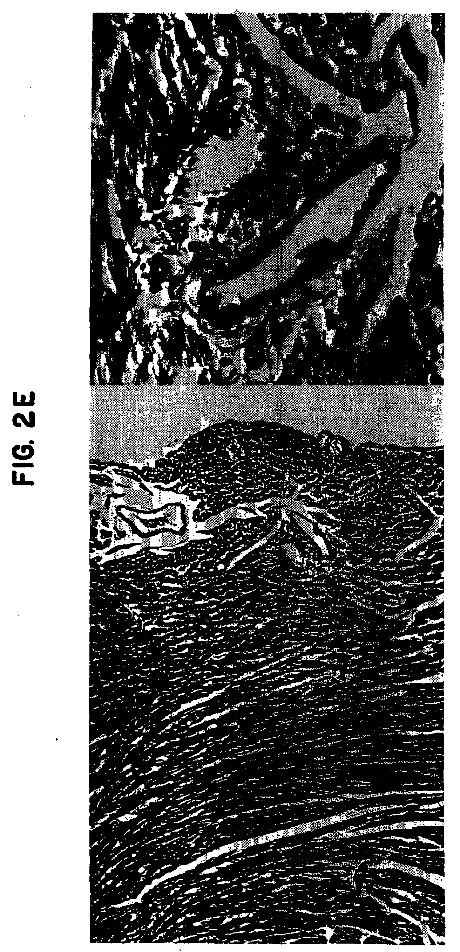

In Figure 2E, normal untransplanted BALB/c hearts are shown.

Similar histologic results were obtained from three allografts in each experimental group. C3H/HeJ (H-2k) recipients treated with CTLA4Ig alone, MR1 alone, or CTLA4Ig/MR1, all showed prolonged survival of BALB/c (H-2d) cardiac allografts when compared to untreated controls (Figure 2A). However, when examined histologically at 58-62 days post-transplant marked differences were apparent.

Allografts from CTLA4Ig-treated recipients showed extensive lymphocytic infiltration, interstitial fibrosis, and severe coronary arterial intimal thickening and fibrosis consistent with chronic rejection (Figure 2B). While the MR1-treated allograft demonstrated less lymphocytic infiltration and interstitial fibrosis, these grafts also had severe coronary vasculopathy characteristic of chronic rejection (Figure 2C).

In marked contrast, the allograft from CTLA4Ig/MR1 treated recipients were remarkably free from lymphocytic infiltration, fibrosis, and most significantly, coronary arterial intimal lesions (Figure 2D). In fact, the parenchyma and blood vessels of these grafts were virtually indistinguishable from those found in normal BALB/c hearts (Figure 2E).

### EXAMPLE 3

This example shows blockade of T cell cytokine and costimulatory molecule transcript expression.

### METHOD

At 8 days after transplantation, the cardiac grafts were removed and total RNA was prepared from tissues using TRIzol Reagent (GIBCO BRL, Gaithersburg, MD). cDNA was synthesized using 5 mg of total RNA template with a Superscript Preamplification System (GIBCO BRL, Gaithersburg, MD) in a final volume of 20 ml. PCR reactions were carried out. PCR products were visualized on ethidium bromide stained 1% agarose (BIO-RAD, Hercules, CA), 2% NuSieve GTG agarose (FMC BioProducts, Rockland, ME) gels. Gel images were stored using a UVP Gel Documentation System 5000. Band intensity was quantified using Gelreader analysis software (National Center for Supercomputing Applications, Urbana, IL).

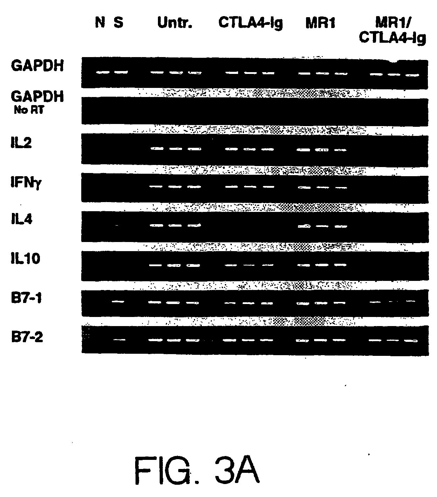

In Figure 3A, intragraft expression of immune mediator transcripts was assessed using RT-PCR in untreated, MR1-treated, CTLA4Ig treated, and MR1/CTLA4Ig treated cardiac allografts.

Three allografts from each treatment group and the control group were analyzed at 8 days post-transplant. Normal heart tissue (N) and a syngeneic heart graft (S) at 8 days after transplantation were included for comparison.

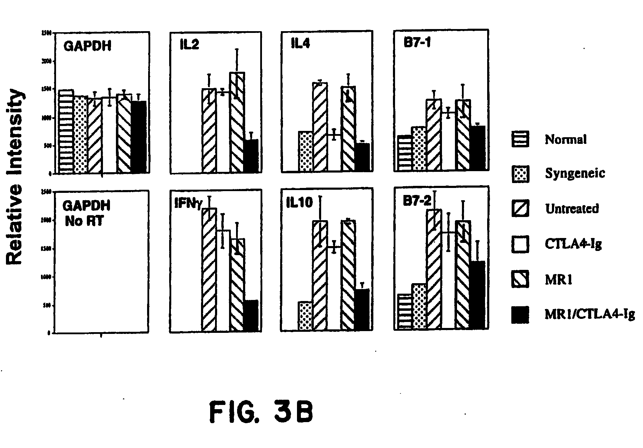

In Figure 3B, graphical representation of the mean PCR product band intensities ± standard deviation are shown.

### DISCUSSION

No consistent differences in the expression of T cell cytokine transcripts for IL-2, IL-4, IL-10, and IFNg or costimulatory molecule transcripts (B7-1, and B7-2) were detectable between the control allografts (Figure 3A, untreated) and MR1-treated allografts (Figure 3A), whereas CTLA4Ig partially inhibited expression of IL-4 transcripts.

Allografts from CTLA4Ig/MR1-treated recipients showed a striking decrease in the expression of both Th1 cytokine (IL-2 and IFNg) and Th2 cytokine (IL-4, and IL-10) transcripts. However, intragraft B7-1 and B7-2 costimulatory molecule transcripts were only modestly reduced in recipients treated with CTLA4Ig/MR1.

PCR reactions using template prepared without reverse transcriptase yielded no products, even for the intron-less GADPH gene (Figure 3A, GADPH, NO RT), confirming the absence of contaminating genomic DNA.

Intragraft B7-1 and B7-2 costimulatory molecule transcripts were only modestly reduced in recipients treated with CTLA4Ig/MR1, (Figure 3) suggesting that CD28/B7-independent or CD40/gp39-independent factors, such as GMCSF (Larsen, C.P., et al. J Immunol 152, 5208-5219 (1994)), may be important regulators of intragraft B7 expression. Thus, MR1-mediated blockade of the CD40 pathway not only inhibits T cell cognate help for effector APC's, but enhances the ability of CTLA4Ig to inhibit T cell activation transcript expression within allografts. These data are consistent with those from our studies in vitro which indicate that while MR1 alone has only a modest negative effect on cellular proliferation in allogeneic mixed leukocyte reactions, it potentiates the inhibitory effects of suboptimal concentrations of CTLA4Ig.

### EXAMPLE 4

This example demonstrates prolongation of murine skin allograft survival using C3H/HeJ mice which received full thickness skin allografts from BALB/c mice.

### METHOD

Segments of either full thickness tail or ear skin of approximately 1 cm square were grafted on to the posterior-lateral thoracic wall of recipient mice and secured in place with a circumferential Bandaid®. The grafts were then followed by daily visual inspection. Rejection was defined as the complete loss of visible epidermal graft tissue. Treatment protocols for MR1 and CTLA4Ig were as detailed for heart transplant recipients in Figure 1. CyA (Sandoz, East Hanover, NJ) at a concentration of 50 mg/ml was administered at a rate of 0.5 ml/hr (~20 mg/kg/day) for 14 days via an osmotic pump (Alzet Model No. 2002, Alza, Palto Alto, CA) which was implanted subcutaneously in the dorsal region of the recipient at the time of skin grafting and removed at 21 days after transplant (Pereira, G.M., Miller, J.F. & Shevach, E.M. J Immunol 144, 2109-2116 (1990)). After sacrifice, the skin graft was excised, formalin fixed and embedded in paraffin. Tissue sections (5 mm) were stained with hematoxylin-eosin.

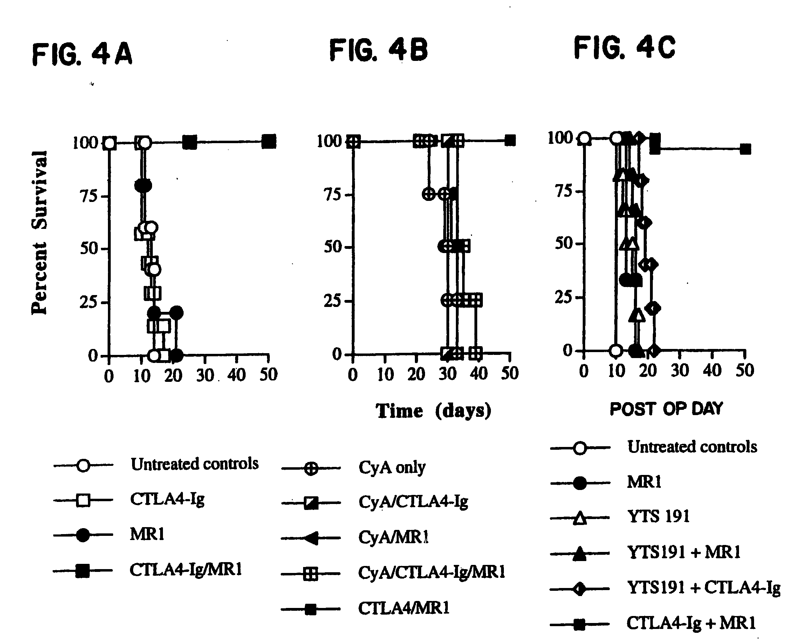

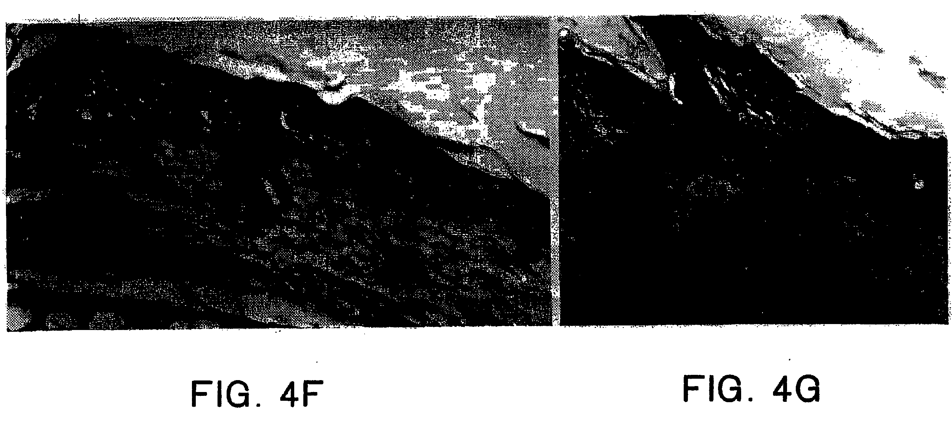

In Figure 4A, C3H/HeJ recipients treated with either MR1 alone (MST = 13 days, n = 5) or CTLA4Ig alone (MST = 12 days, n = 7) rejected fully MHC-disparate BALB/c skin grafts at the same rate as an untreated control group (MST = 13 days, n = 5). In contrast, when MR1 and CTLA4Ig were administered together in the perioperative period, the allografts enjoyed markedly prolonged survival (MST >50, n = 15).

In Figure 4B, mice treated with CyA alone (MST = 30 days, n = 4), CyA plus CTLA4Ig (MST = 30 days, n = 5), or CyA and MR1 (MST = 32 days, n = 4) all displayed similar modestly prolonged skin graft survival. Surprisingly, the salutary effect of CTLA4Ig/MR1 on skin graft survival was abolished by concomitant cyclosporine administration (MST = 34 days, n = 4).

In Figure 4C, C3H recipients of BALB/c skin grafts were not treated (MST 10d, n=3), or treated with MRI (MST 13d, n=3), YTS191.1 (MST 14d, n=6), YTS191.1 and MR1 (MST 16d, n=6), YTS191.1 and CTLA4Ig (MST 19d, n=5), or CTLA4Ig and MR1 (MST > 50d, n=22). Thus far, >53 mice have been treated with CTLA4Ig/MR1. Of these, 2 died on days 13 and 21. All others have remained healthy throughout the experiments without signs of weight loss, infection, or malignancy.

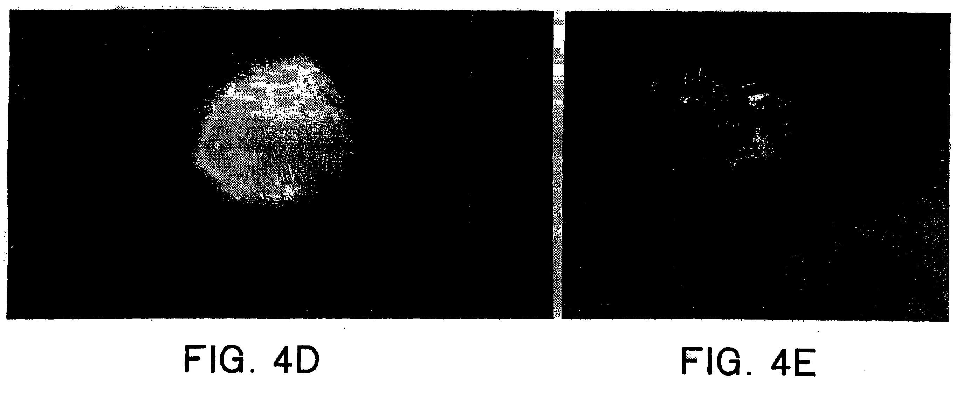

Healthy appearance of a BALB/c skin graft on an CTLA4Ig/MR1 treated C3H/HeJ recipient at 50 days after transplant (Figure 4D), contrasts sharply with a control allograft undergoing rejection (Figure 4E). On hematoxylin-eosin stained sections the accepted graft at 100 days after transplant demonstrated well preserved epidermis, hair follicles and adnexal structures (Figure 4F), which is in contrast to a BALB/c skin graft on an untreated C3H/HeJ recipient 8 days after transplant which shows an extensive lymphocytic infiltrate (Figure 4G).

### DISCUSSION

The effects of CTLA4Ig and MR1, alone and in combination on primary skin allograft survival in mice were tested (Figure 4). For comparison, recipients were also treated with CyA alone or CyA combined with either CTLA4Ig or MR1. C3H/HeJ recipients treated with either MR1 alone or CTLA4Ig alone rejected fully MHC-disparate BALB/c skin grafts at the same rate as untreated controls (Figure 4A).

Mice treated with CyA alone, CyA plus CTLA4Ig, or CyA and MR1 all displayed modestly prolonged skin graft survival (Figure 4B). However, all of these allografts were ultimately rejected with no apparent effects between either drug and CyA.

As a rigorous test of the ability of CD40/CD28 blockade to interrupt alloimmune responses, we studied the effects of perioperative treatment with CTLA4Ig and MR1, alone and in combination on primary skin allograft survival in mice. For comparison, recipients were also treated with CyA, an anti-CD4 mAb YTS191.1, or with either of these agents combined with CTLA4Ig or MR1 (Figures 4B and 4C). C3H/HeJ recipients treated with either MR1, CTLA4Ig, or YTS191.1 alone rejected fully MHC-disparate BALB/c skin grafts at essentially the same rate as untreated controls (Figures 4B and 4C). Mice treated with YTS191.1 and MR1, YTS191.1 and CTLA4Ig, CyA alone, CyA plus CTLA4Ig, or CyA and MR1 displayed modestly prolonged skin graft survival (Figures 4B and 4C). However, all of these allografts were ultimately rejected.

In contrast, on recipients treated with both MR1 and CTLA4Ig in the perioperative period, the skin allografts demonstrated markedly prolonged survival. Visual examination of these allografts at 50 days after transplantation showed the grafts to be healthy in appearance, well vascularized, supple, and bearing short white hair (Figure 4D). Histologically, the accepted grafts demonstrated well preserved epidermis, hair follicles and adnexal structures (Figure 4F). Surprisingly, the salutary effect of CTLA4Ig/MR1 on skin graft survival was abolished by concomitant cyclosporine administration (Figure 4B).

The remarkable potency of this effect was most clearly evident in the primary skin allograft model. Neither CTLA4Ig or MR1 alone or with CyA significantly prolonged skin allograft survival. Only the combination of CTLA4Ig and MR1 produced >50 day survival of fully-MHC mismatched skin allografts. Similar prolongation in this stringent test of inhibition of the alloimmune response has previously only been observed using vigorous cytoablative and/or hematopoietic chimerism-based strategies (Mayumi, H. & Good, R.A. J Exp Med 169, 213-238 (1990); Ildstad, S.T. & Sachs, D.H., Nature 307, 168-170 (1984); Ilstad, S.T., et al. J Exp Med 162, 231-44 (1985); Cobbold, S.P., Martin, G., et al. Nature 323, 164-166 (1986); Qin, S., et al. Science 259, 974-977 (1993)).

### EXAMPLE 5

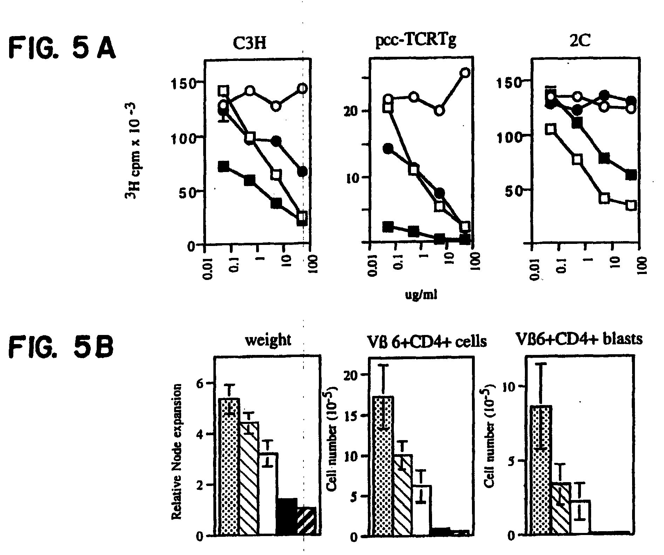

To explore the effect of blockade of the CD28 and CD40 pathways on T cell proliferation, we studied the primary allogeneic mixed leukocyte reaction using T cells from both lek-restricted pigeon cytochrome c-reactive (pcc-TCRTg) and Ld-alloreactive (2C) T cell receptor transgenic mice (REF HED and LOH). CTLA4Ig, a fusion protein which binds to the ligands for CD28 and its homologue CTLA4, effectively inhibited proliferation of all three T cell populations (Figure 5A).

In contrast, blockade of the CD40 pathway with the hamster anti-gp39 mAb, MR1, modestly (~50%) inhibited the proliferation of C3H/HeJ T cells responding to BALB/c dendritic cells, dramatically inhibited (³85%) pcc-TCRTg T cells to reacting to cytochrome c, but had negligible effects on the proliferation of 2C T cells responding to Ld-bearing BALB/c dendritic cells (Figure 5A).

Furthermore, simultaneous blockade with these agents cooperatively inhibited T cell proliferation in allogeneic mixed leukocyte reactions and pcc-TCRTg T cells, whereas MR-1 had no effect or slightly augmented the proliferation of 2C T cells when combined with CTLA4Ig (Figure 5A). These results indicate that not all T cells are dependent on CD40 signals for clonal expansion and may explain the inability of CD40 blockade to completely inhibit allograft rejection.

The effect of CD28 and CD40 blockade on T cell responses in vivo was assessed in C3H/HeJ (H-2k, MMTV-7-) immunized with DBA/2(H-2d, MMTV-7+) splenocytes in their foot pads. Five days after immunization the draining popliteal lymph nodes in control mice treated with human IgG demonstrated a 4-6 fold increase in weight (Figure 5B). This was accompanied by a 30 fold expansion in the number of MMTV-7 superantigen-reactive Vb+CD+4 T cells and a >90 fold increase in the number of Vb+CD+T cell blasts within the popliteal lymph node. Alone, CTLA4Ig or MR1 partially inhibited these responses. In contrast, the combination of CTLA4Ig/MR1 essentially ablated the increase in lymph node size and the expansion and blastogenesis of Vb+CD4+T cells (Figure 5B).

These data show that simultaneous blockade of the CD28 and CD40 pathways inhibit complex T-dependent immune responses in vitro and in vivo.

### EXAMPLE 6

This example shows that simultaneous blockade of the CD28 and CD40 pathways produces marked inhibition of both the cellular and antibody response to xenoantigen and long-term acceptance of xenogeneic (rat to mouse) cardiac and skin grafts without the need for a cytoablative conditioning therapy.

### METHODS

LYMPH NODE ASSAY. Male C3H (Jackson Laboratory, Bar Harbor, ME) mice were immunized with 2 x 106 male Sprague-Dawley (Harlan, Indianapolis, IN) irradiated (2000 RADS) rat splenocytes in 50 µl of sterile normal saline in the left foot pad and 50 µl of sterile normal saline in the right foot pad and then treated intraperitoneally (i.p.) with MR1 (500 µg), CTLA4-Ig (500 µg) or both reagents (500 µg each) on days 0, 2 and 4. On day 5, the popliteal lymph nodes were removed, weighed and then teased apart, washed, before resuspension in 600 µl of RPMI 1640 with 10% FBS (Mediatech, Herndon, VA). Each resuspended node was then divided into four equal aliquots (150 µl each). Three of the aliquots were plated into a 96 well plate. 3H-thymidine (1 µCi/well) (Amersham, Arlington Heights, IL) incorporation was measured after 24 hours incubation at 37 oC. The results for each individual animal therefore represent the mean of the 3 wells per node. The fourth aliquot was incubated for 24 hours at 37 oC and served as the source of supernatant for cytokine analysis with ELISA. Each point on all of the graphs represents the mean ± standard deviation of 5 mice per group. The experiment was repeated with similar results.

CYTOKINE ELISA. Sandwich ELISA was performed using paired antibodies {anti-IL-2, anti-IFN-gamma, anti-IL-2 biotin, anti-IL-4 biotin, anti-IFN-gamma biotin (Pharmingen, San Diego, CA), anti IL4 (kind gift from Peter Jensen)} and streptavidin-HRP (Pierce, Rockford, IL). Colorimetric detection was assayed using TMB substrate (Pierce). Data were collected using a SpectraMax plate reader and plotted as absorbance (370 nm) +/- sem. Standard curves for each cytokine were generated using recombinant cytokine (rIL2, Boehringer Mannheim, Indianapolis, IN; rIL4, R&D Systems, Minneapolis, MN; and rIFN-gamma, Biosource International, Camarillo, CA).

MICE. Male C3H/HeJ (H-2k) and DBA/2 (H-2d) mice and Sprague-Dawley rats were purchased from The Jackson Laboratory (Bar Harbor, ME) and used at 8-12 weeks of age.

CARDIAC TRANSPLANTATION. C3H/HeJ or DBA mice were transplanted with primarily vascularized Sprague-Dawley rat heart xenografts and monitored for rejection as described in Larsen C.P., Alexander D.Z., Hollenbaugh D., et al., Transplantation, 61(1):4-9 (1996) and Corry R.J., Winn H.J., Russell P.S., Transplantation, 16(4):343-350 (1973). Recipients were treated with 500 mg CTLA4-Ig combined with 500 mg MR1 on days 0, 2, 4 and 6. Control groups included recipients treated with CTLA4-Ig alone, MR1 alone or Human Ig. Paraffin embedded tissue sections (5 µm) were stained with Masson's Trichrome or hematoxylin-eosin. Histologic specimens were reviewed by a cardiac transplant pathologist (KJW) blinded to the treatment modality.

SKIN TRANSPLANTATION. Full thickness skin grafts (~ 1 cm2) from Sprague-Dawley rats were transplanted on the dorsal thorax of C3H recipient mice and survival followed by daily visual inspection. Rejection was defined as the complete loss of visible epidermal graft tissue. Control groups included recipients treated with: CTLA4-Ig alone; MR1 alone; and Human Ig. Two additional mice in each experimental group were sacrificed at 20 days post transplant for histologic analysis. XENOANTIBODY ASSAY. Serum was collected via tail bleed from anesthetized animals. Single cell suspensions from lymph nodes of a Sprague-Dawley rat were used as target cells, and incubated with recipient mouse serum for 20 minutes at 4oC. The cells were washed and IgG xenoantibodies were detected with donkey anti-mouse IgG Biotin (Jackson ImmunoResearch, West Grove, PA) followed by streptavidin-PE (Southern Biotech, Birmingham, AL). Cells were analyzed on a Becton-Dickinson FACscan using Cellquest Software.

### RESULTS

As an initial approach to determine the effects of CD28 and CD40 blockade on responses to xenoantigenic challenge in vivo, we used the popliteal lymph node assay as described in Larsen C.P., Elwood E.T., Alexander D.Z., et al., Nature, 381:434-438 (1996). C3H/HeJ (H-2k) mice were injected with irradiated Sprague-Dawley (SD) rat splenocytes. Five days after foot pad immunization, the draining popliteal lymph nodes in control mice treated with human IgG demonstrated a 5.2 fold increase in weight relative to the contralateral node which was inoculated with sterile saline (Fig. 6A). CTLA4-Ig partially inhibited nodal expansion. Similarly MR1 partially inhibited this response. In contrast, the combination of CTLA4-Ig/MR1 essentially ablated xenoantigen-induced lymph node expansion (Fig. 6A).

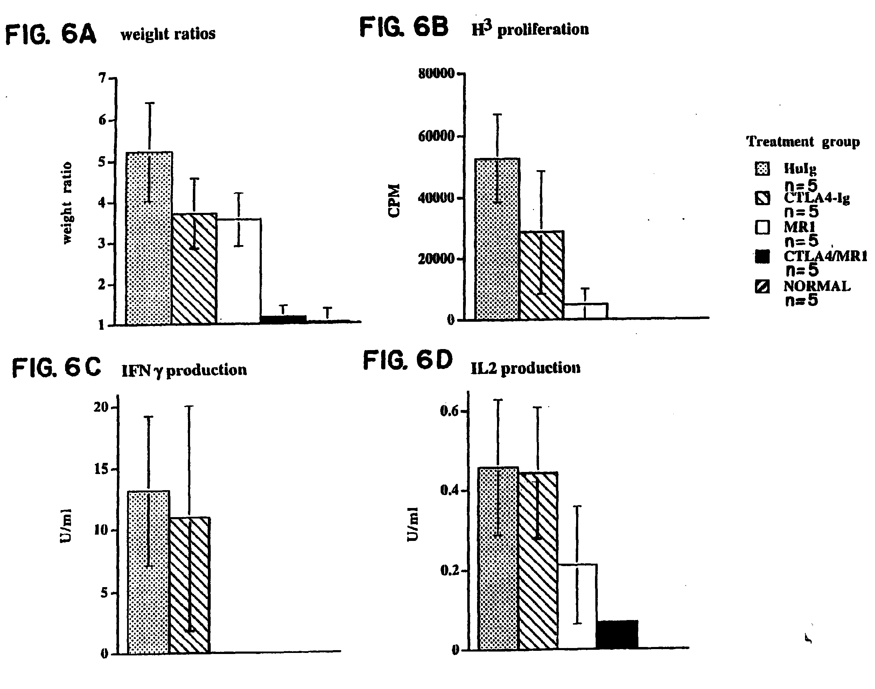

We then compared the ex vivo proliferation of lymph node cells from the different groups of xenoantigen-primed mice. While either treatment alone only partially blocked proliferation (Fig.6B), the combination of CTLA4-Ig/MR1 essentially ablated the proliferative response (302+/-235 CPM for the combination versus 143+/-145 for a normal unstimulated node). Furthermore, the combination therapy markedly suppressed Th1 cytokines (IL-2 and IFNg) to the level of normal unstimulated cells (Figs. 6C and 6D). Levels of the Th2 cytokine IL4 were below the level of detection in all samples.

It is important to note that this potent immune modulation is not the result of cellular deletion. Flow cytometric analysis of the peripheral blood of treated mice showed no depletion of CD4+ or CD8+ T cells, B cells, or NK cells. These data are the result of an individual analysis of 3 mice per group treated with either CTLA4-Ig or MR1 alone or the combination of these agents on days 0, 2, 4, and 6 as described in the methods section. Peripheral blood was collected by tail bleed on days 6 and 20.

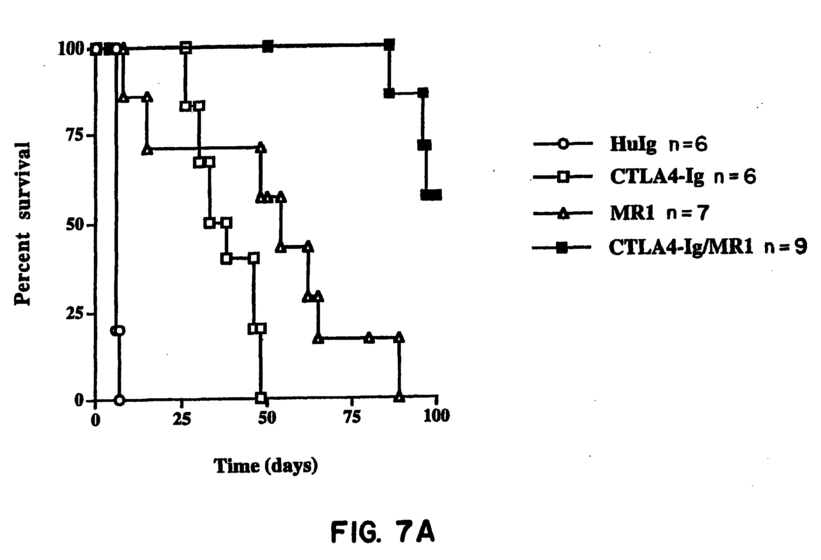

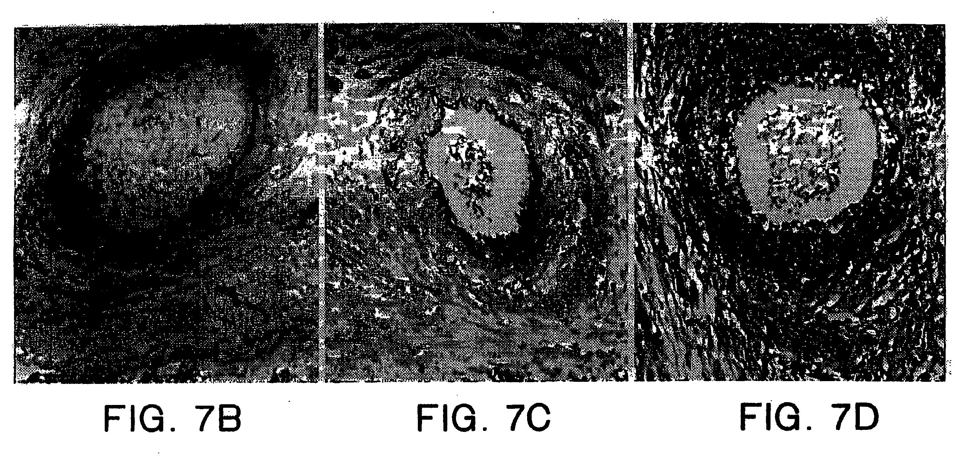

The results of the lymph node assays suggested that simultaneous blockade of the B7/CD28 and CD40/gp39 pathways would inhibit xenograft rejection. To explore this hypothesis we studied a vascularized cardiac xenograft model using Sprague-Dawley rats as donors and C3H/HeJ mice as recipients. Treatment with either CTLA4-Ig (MST=33 days) or MR1 (MST=51 days) alone prolonged xenograft survival when compared to untreated controls (MST=6 days) (Fig. 7A). However, CTLA4-Ig/MR1 in combination markedly prolonged survival (MST=104.5 days).

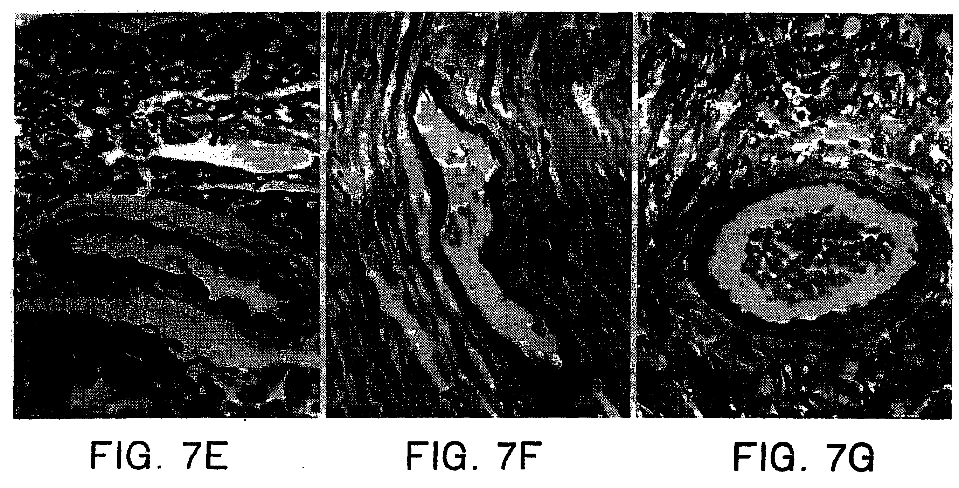

When examined histologically at 20 days post-transplant, xenografts treated with either CTLA4-Ig alone (Fig. 7C) or MR1 alone (Fig. 7D) showed heavy lymphocytic infiltration with evidence of myocyte destruction and vasculopathy consistent with moderate to severe cellular rejection. In sharp contrast, the xenografts from CTLA4-Ig/MR1 treated recipients were essentially free from lymphocytic infiltration, interstitial fibrosis, and coronary arterial intimal lesions (Fig. 7F). CTLA4-Ig/MR1-treated cardiac xenografts demonstrated excellent preservation of both myocytes and vascular structures at day 122 (Fig. 7G). Untreated xenografts showed widespread tissue destruction at day 6 (Fig. 7B). A normal untransplanted Sprague-Dawley rat heart is shown in Fig. 7E.

As a more stringent test of the ability of CD40/CD28 blockade to inhibit xenogeneic immune responses, we studied the effects of short term CD28 and /or CD40 blockade, on primary skin xenograft survival in mice.

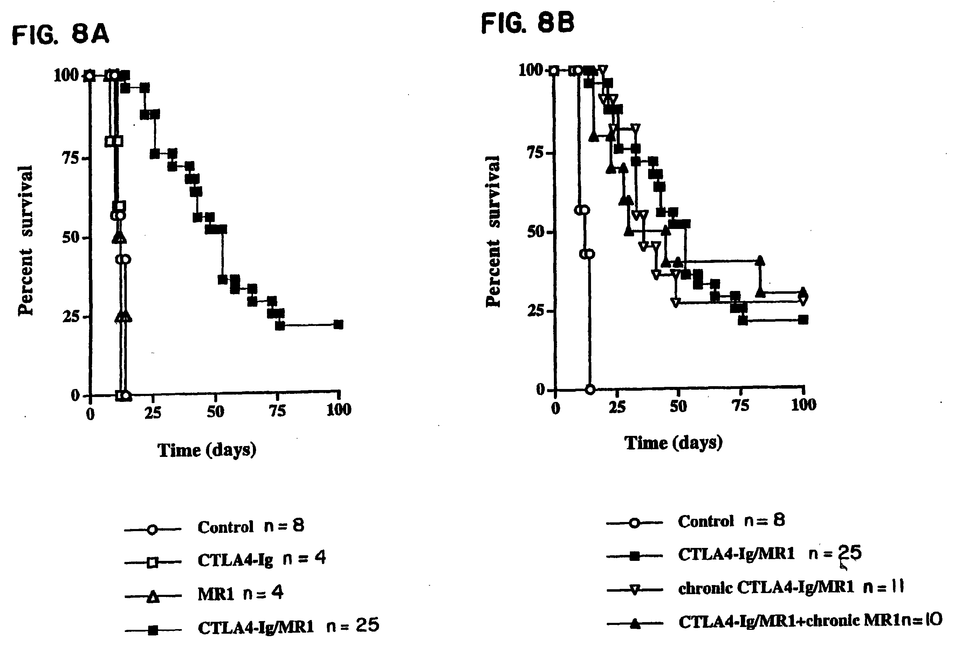

C3H recipients treated with either MR1 (MST=11.5 days n=4) or CTLA4-Ig (MST=12 days n=4) alone rejected full thickness skin grafts from Sprague-Dawley rats at the same rate as untreated controls (untreated controls MST=11.5 days n=8) (Fig. 8A). In contrast, the skin xenografts on recipients treated with simultaneous MR1 and CTLA4-Ig in the perioperative period, demonstrated markedly prolonged survival (MST=53 days n=25) (Fig. 8A). A total of 25 mice received xenografts and treatment with CTLA4-Ig/MR1. With the exception of one mouse that died on day 4, all others have remained healthy throughout the experiments without signs of weight loss, infection, or malignancy.

Chronic treatment (beginning after the standard 4 dose regimen) with either the CTLA4-Ig/MR1 combination (500 µg of both agents weekly until day 100 or rejection, whichever came first) or MR1 (500 µg of MR1 weekly until day 100 or rejection) resulted in no significant change in xenograft survival (Fig. 8B).

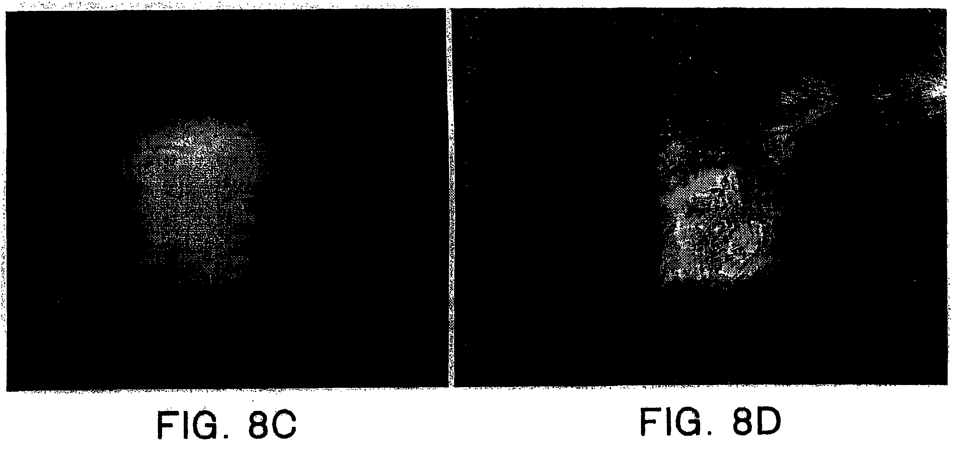

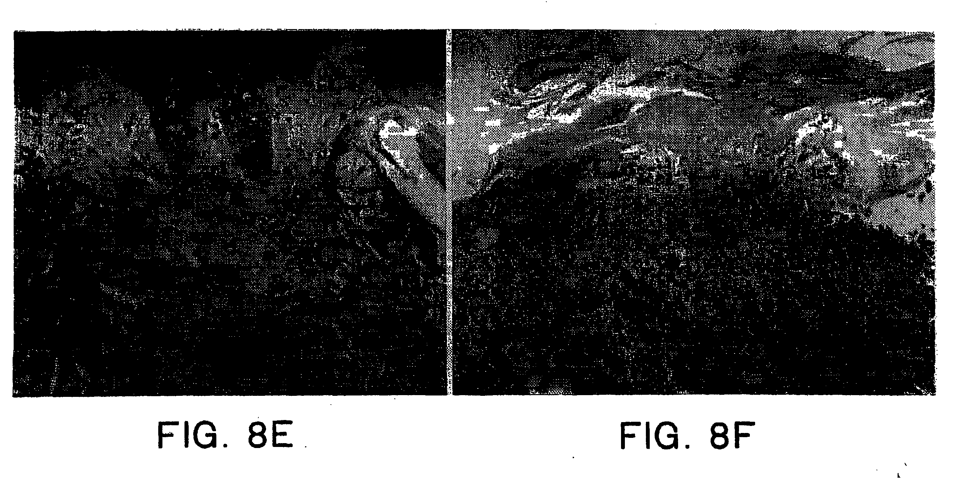

Xenograft survival after simultaneous MR1/CTLA4-Ig therapy was 52% and 21% at 50 and 100 days respectively. The xenografts surviving at 50 (Fig. 8E) and 100 (Fig. 8C) days after transplantation were healthy in appearance and demonstrated well preserved histologic architecture. In untreated controls without the combination therapy, rejection was prompt and these xenografts showed marked inflammatory infiltrates (Figs. 8D and 8F).

Similar prolongation of Sprague-Dawley skin xenografts was also observed in DBA[H-2d]) recipients (untreated controls MST=14 days (n=5) versus CTLA4-Ig/MR1 MST=86 days n=5), suggesting that the potent effect of the combination treatment is not limited to a single recipient mouse strain.

The progressive loss of skin xenografts between 25 and 75 days post-transplant suggested that late graft failure might be due to subtherapeutic concentrations of CTLA4-Ig and/or MR1. To address this possibility, after the standard four-dose combination regimen mice were treated weekly with either the CTLA4-Ig/MR1 combination or MRI alone for 100 days or until graft loss occurred. Neither of these chronic therapy strategies appreciably improved skin xenograft survival, suggesting that graft failure in CTLA4-Ig/MR1 treated mice results from factors other than inadequate drug titers and that alternate pathways not completely inhibited by CTLA4-Ig/MR1 may be important in sub-acute xenograft loss.

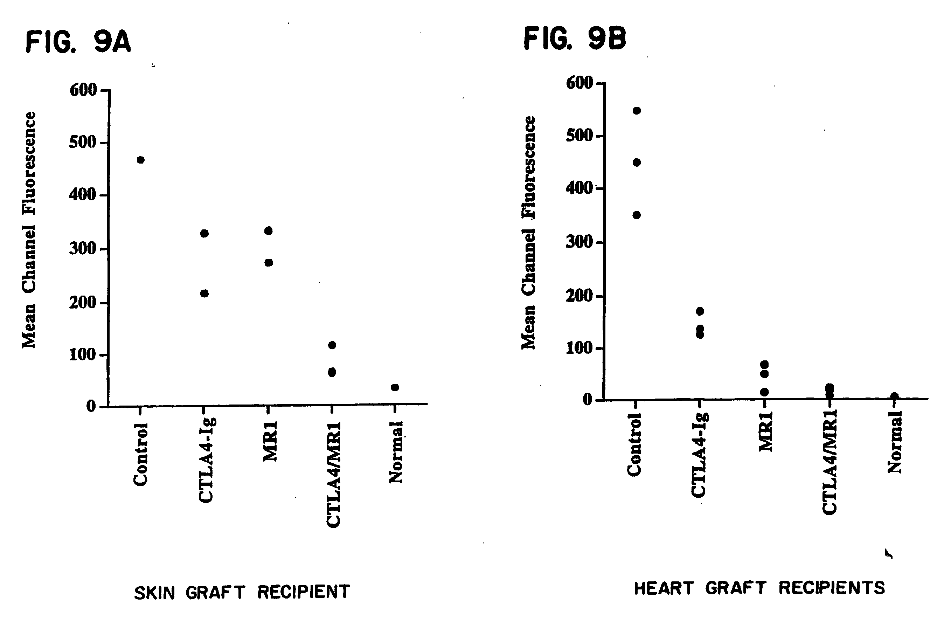

In addition to cell-mediated effector mechanisms, evoked xenoantibody responses may play an important role in accelerated vascular rejection of concordant xenografts, Aksentijevich I., Sachs D.H., Sykes M., Transplantation, 53(5):1108-14 (1992). To test the effect of simultaneous blockade on evoked xenoantibody responses, serum samples from C3H/HeJ mice were analyzed for anti-rat antibodies at 55 days after receiving a Sprague-Dawley rat skin graft (Fig. 9A) and at 20 days after receiving a Sprague-Dawley rat heart graft (Fig. 9B). Treatment with either CTLA4-Ig or MR1 alone decreased the IgG antibody response, whereas the simultaneous combination CD28/CD40 blockade essentially eliminated the evoked antibody response to rat xenoantigens. Thus, inhibition of both T cell activation and antibody production could be functionally important in xenograft survival after simultaneous blockade of the B7/CD28 and CD40/gp39 pathways.

### DISCUSSION

Combined blockade of the CD28 and CD40 pathways markedly inhibits the immune response to xenoantigen. The potency of this combination therapy was particularly demonstrated in the primary skin xenograft model. Neither agent alone prolonged skin xenograft survival, while, in contrast, the simultaneous combination of CTLA4-Ig and MR1 cooperatively inhibited xenograft rejection. The uniqueness of the findings resides in the stringency of the skin graft model, as CTLA4-Ig alone has previously been shown to prolong the survival of xenogeneic pancreatic islets in a mouse model, Lenschow D., Zeng Y., Thistlethwaite J., et al., Science, 257:789-792 (1992). Long-term survival of xenogenic skin grafts have previously only been observed using vigorous cytoablative and/or hematopoeitic chimerism-based strategies, Ildstad S.T., Sachs D.H., Nature, 307:168-170 (1984), Zhao Y., Swenson K., Sergio J., Am J.S., Sachs D.H., Sykes M., Nat. Med., 2(11):1211-1216 (1996), Mayumi H., Good R.A., J. Exp. Med., 169(1):213-238 (1990), Cobbold S.P., Martin G., Zin S., Waldman H., Nature, 323:164-166 (1986), Qin S., Cobbold S., Benjamin R., Waldmann H., J. Exp. Med., 169:779-794 (1989).

The observation that simultaneous CD28/CD40 blockade can dramatically prolong xenograft survival suggests that both the antibody and cell mediated mechanisms for destruction of the xenograft may be effectively inhibited by this strategy. While the etiology of acute vacular xenograft rejection remains to be completely defined, there is evidence that it is caused, at least in part, by the development of xenoreactive antibodies (Cotterell A.H., Collins B.H., Parker W., Harland R.C., Platt J.L, Transplantation, 60(8):861-868 (1995)). The rapid destruction of untreated control cardiac xenografts in our model, in the absence of a cellular infiltrate, suggests a role for antibody mediated rejection. This observation and those of others (Aksentijevich I., Sachs D.H., Sykes M., Transplantation, 53(5):1108-14 (1992)), combined with the documented dramatic inhibition of the evoked xenoantibody response after blockade of the CD28 and CD40 pathways (Figures 9A and 9B), supports the hypothesis that xenoresponses may be sufficiently controlled by inhibition of these pathways to permit the development of non-cytoablative strategies for xenotransplantation in discordant species combinations.

While combined blockade of the CD28 and CD40 pathways markedly inhibited the xenograft rejection response, this blockade did not achieve uniform indefinite cardiac or skin graft survival in our experimental system. The observation that prolonged treatment did not improve graft survival was surprising. This suggests that inadequate blockade of these pathways is not the cause for "late" graft failure and raises the possibility that alternate pathways such as NK cells or other cells, which do not require CD28/CD40 co-stimulation, may promote late xenograft rejection. Suggestive evidence for the contribution of NK cells to xenograft rejection support this possibility, Zhao Y., Swenson K., Sergio J., Am J.S., Sachs D.H., Sykes M., Nat. Med., 2(11):1211-1216 (1996), Malyguine A.M., Saadi S., Platt J.L., Dawson J.R., Transplantation, 61(1):161-164 (1996). In addition, we have shown that the inhibition of the rejection of concordant heart and skin xenografts by the simultaneous blockade of the CD40 and CD28 pathways is associated with the prevention of the "late" development of the evoked xenoreactive antibody responses (Figures 9A and 9B), we have not excluded the possibility that the development of an antibody response may be associated with delayed graft loss.

The ability of combined CTLA4-Ig/MR1 treatment to block the development of transplant vasculopathy in cardiac xenografts and prolong skin xenografts is of significant clinical relevance. The refinement of techniques to inhibit the effect of natural preformed xenoreactive antibodies combined with further study of CD28 and CD40 pathway blockade promises the possibility of effective new strategies to facilitate clinical xenograft transplantation.

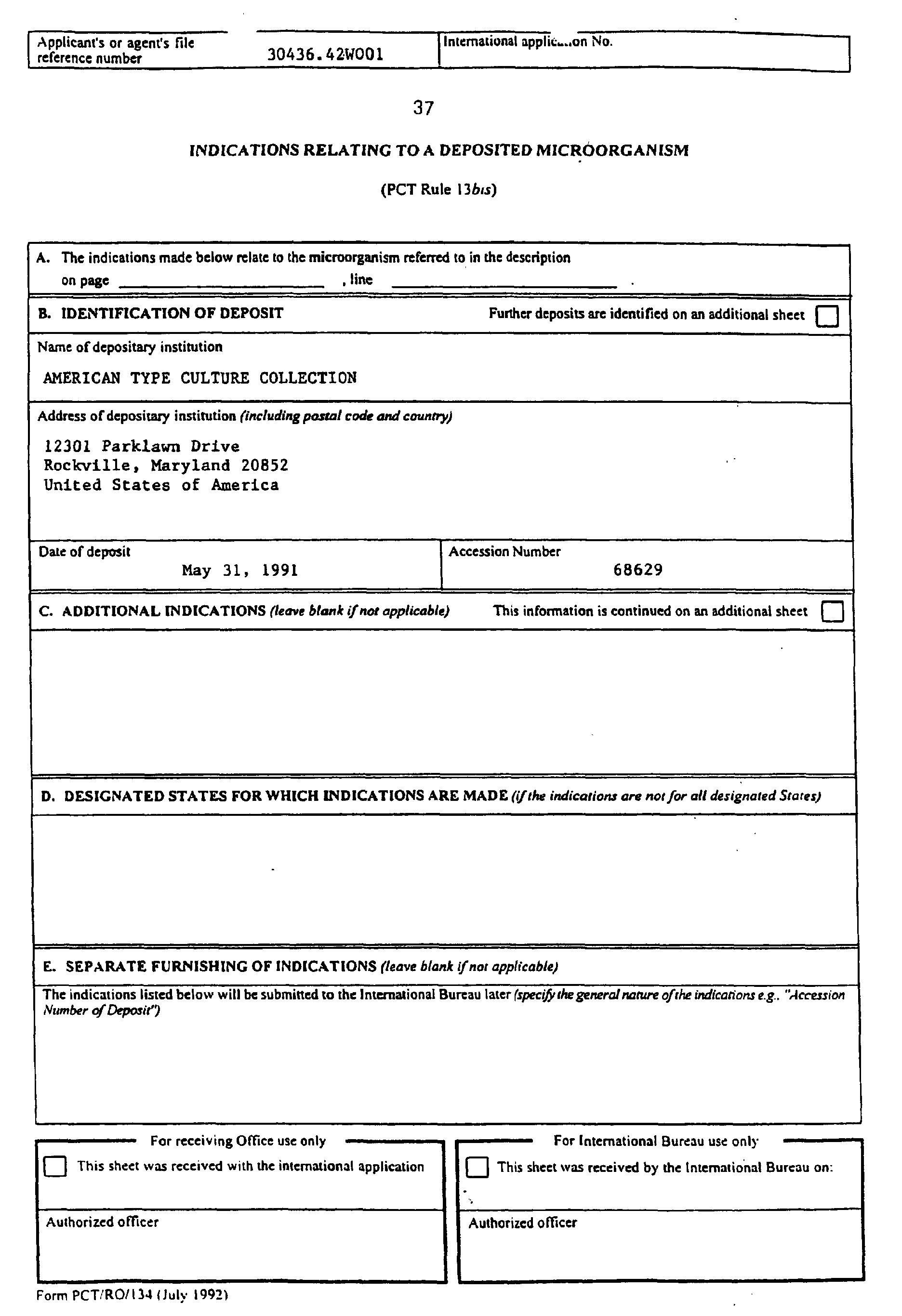

## Claims

1. A pharmaceutical composition useful to inhibit an immune response comprising an effective amount of a combination of (a) at least one soluble ligand which recognizes and binds any one of CTLA4, CD28, and B7 antigens, and (b) at least one soluble ligand which recognizes and binds anyone of gp39 and CD40 antigens and an acceptable carrier.

2. The pharmaceutical composition of claim 1, wherein a first soluble ligand recognizes and binds a B7 antigen and a second soluble ligand recognizes and binds a gp39 antigen.

3. The pharmaceutical composition of claim 1, wherein a first soluble ligand recognizes and binds a B7 antigen and a second soluble ligand recognizes and binds a CD40 antigen.

4. The pharmaceutical composition of claim 1, wherein a first soluble ligand recognizes and binds a CD28 antigen and a second soluble ligand recognizes and binds a gp39 antigen.

5. The pharmaceutical composition of claim 1, wherein a first soluble ligand recognizes and binds a CD28 antigen and a second soluble ligand recognizes and binds a CD40 antigen.

6. The pharmaceutical composition of claim 1, wherein a first soluble ligand recognizes and binds a CTLA4 antigen and a second soluble ligand recognizes and binds a gp39 antigen.

7. The pharmaceutical composition of claim 1, wherein a first soluble ligand recognizes and binds a CTLA4 antigen and a second soluble ligand recognizes and binds a CD40 antigen.

8. The pharmaceutical composition of claims 1, 2 or 3, wherein the soluble ligand which recognizes and binds the B7 antigen is an antibody reactive with B7 antigen, a soluble CTLA4, or a soluble CD28.

9. The pharmaceutical composition of claim 8, wherein the antibody reactive with B7 antigen is anti-BB1 monoclonal antibody.

10. The pharmaceutical composition of claim 8, wherein the soluble CTLA4 is a recombinant binding molecule having at least a portion of the extracellular domain of CTLA4.

11. The pharmaceutical composition of claim 10, wherein the extracellular domain of CTLA4 is joined to a non-CTLA4 protein sequence.

12. The pharmaceutical composition of claim 11, wherein the non-CTLA4 protein sequence is at least a portion of an immunoglobulin molecule.

13. The pharmaceutical composition of claim 8 or 10, wherein the soluble CTLA4 is CTLA4Ig fusion protein.

14. The pharmaceutical composition of claim 13, wherein the CTLA4Ig fusion protein is CTLA4Ig designated ATCC 68629.

15. The pharmaceutical composition of claim 8 or 10, wherein the soluble CTLA4 is a CD28lg/CTLA4lg fusion protein hybrid.

16. The pharmaceutical composition of claim 15, wherein the CD28Ig/CTLA4Ig fusion protein hybrid comprises a first amino acid sequence corresponding to a portion of the extracellular domain of CD28 receptor fused to a second amino acid sequence corresponding to a portion of the extracellular domain of CTLA4 receptor and a third amino acid sequence corresponding to the hinge, CH2 and CH3 regions of human immunoglobulin C(gamma)1.

17. The pharmaceutical composition of claim 8, wherein the soluble CD28 is a recombinant binding molecule having at least a portion of the extracellular domain of CD28.

18. The pharmaceutical composition of claim 8 or 17, wherein the soluble CD28 is a CD28lg/CTLA4lg fusion protein hybrid.

19. The pharmaceutical composition of claims 1, 4 or 5, wherein the soluble ligand which recognizes and binds the CD28 antigen is an antibody reactive with CD28 or a soluble B7.

20. The pharmaceutical composition of claims 1, 6 or 7, wherein the soluble ligand which recognizes and binds the CTLA4 antigen is an antibody reactive with CTLA4 or a soluble B7.

21. The pharmaceutical composition of claims 1, 2, 4 or 6, wherein the soluble ligand which recognizes and binds the gp39 antigen is an antibody reactive with the gp39 antigen or a soluble CD40.

22. The pharmaceutical composition according to claim 21, wherein the antibody reactive with the gp39 antigen is MR1 monoclonal antibody.

23. The pharmaceutical composition according to claim 21, wherein the antibody reactive with the gp39 antigen is anti-human gp39 antibody.

24. The pharmaceutical composition of claims 1, 3, 5 or 7, wherein the soluble ligand which recognizes and binds the CD40 antigen is an antibody reactive with CD40 or a soluble gp39.
